# FASCICULE DE BREVET EUROPEEN

(11) **EP 2 121 917 B1**
(45) Date de publication et mention de la délivrance du brevet: **26.02.2014**
(21) Numéro de dépôt: 07872428.3
(22) Date de dépôt: 20.12.2007
(51) Int. Cl.: C12N 15/09

(54) **LIGNEE CELLULAIRE A FORTE ACTIVITE TRANSCRIPTIONNELLE POUR LA PRODUCTION DE PROTEINES, NOTAMMENT THERAPEUTIQUES**
ZELLLINIE MIT EINER HOHEN TRANSKRIPTIONSAKTIVITÄT ZUR HERSTELLUNG VON PROTEINEN, INSBESONDERE VON THERAPEUTISCHEN PROTEINEN
CELL LINE HAVING A HIGH TRANSCRIPTION ACTIVITY FOR THE PRODUCTION OF PROTEINS, IN PARTICULAR THERAPEUTIC PROTEINS

(30) Priorité: 20.12.2006 FR 0611157
(43) Date de publication de la demande: 25.11.2009
(73) Titulaire: Laboratoire Français du Fractionnement et des Biotechnologies, 91940 Les Ulis (FR)
(72) Inventeur: GAUCHER, Christine, F-59320 Sequedin (FR); SIRAC, Christophe, F-19100 Brive (FR)
(74) Mandataire: Hirsch & Associés
(86) Numéro de dépôt international: PCT/FR2007/002144
(87) Numéro de publication internationale: WO 2008/096070

(56) Documents cités:
- WO-A-2004/061104
- ARAKI K ET AL: "EXCHANGEABLE GENE TRAP USING THE CRE/MUTATED LOX SYSTEM" MOLECULAR AND CELLULAR BIOLOGY, AMERICAN SOCIETY FOR MICROBIOLOGY, WASHINGTON, US, vol. 45, no. 5, juillet 1999 (1999-07), pages 737-750, XP000877476 ISSN: 0270-7306
- HOUDEBINE L-M: "THE METHODS TO GENERATE TRANSGENIC ANIMALS AND TO CONTROL TRANSGENE EXPRESSION" BRAUWELT, NUERNBERG, DE, vol. 98, no. 2/3, 25 septembre 2002 (2002-09-25), pages 145-160, XP001147895 ISSN: 0724-696X
- HAUSER H ET AL: "NEW APPROACHES TOWARDS EX VIVO AND IN VIVO GENE THERAPY" CELLS TISSUES ORGANS, KARGER, BASEL, CH, vol. 167, no. 2/3, 2000, pages 75-80, XP000961432 ISSN: 1422-6405

## Description

La présente invention concerne un procédé d'obtention d'une lignée cellulaire fortement productrice d'une protéine d'intérêt comprenant en particulier l'intégration d'une séquence codant une protéine d'intérêt dans un site de forte activité transcriptionnelle pour la production de protéines, notamment thérapeutiques, ainsi que les lignées cellulaires obtenues par ce procédé.

### Domaine de l'invention

Grâce au développement des biotechnologies la production de protéines recombinantes représente actuellement un secteur d'activité majeur dans le domaine biomédical avec de multiples applications diagnostiques ou thérapeutiques. En effet, l'évolution des techniques d'ingénierie moléculaire et cellulaire offre à présent la possibilité de maîtriser la production de quantités importantes de protéines recombinantes dans différentes cellules hôte d'expression.

Différents systèmes biologiques peuvent être utilisés pour la production de protéines recombinantes : les bactéries (1, 2), les champignons/levures (3-5), les végétaux (6), les cellules d'insecte (7, 8) ou les cellules de mammifère (9-12). Toutefois, les cellules de mammifères sont les plus fréquemment utilisées pour la production de protéines recombinantes complexes à usage thérapeutique (tels que les anticorps monoclonaux) en raison d'une meilleure capacité post-traductionnelle à assembler, à glycosyler et à modifier les protéines recombinantes synthétisées (10, 11).

Malheureusement, le niveau naturel de productivité des cellules de mammifères est faible lorsqu'on le compare aux niveaux d'expression obtenus dans les bactéries ou les levures. En effet, l'étape d'intégration du vecteur d'expression dans le génome de la cellule hôte représente un événement rare (1/10 000 selon Gorman and Bullock 2000, réf. 13), au cours duquel le site d'insertion, le nombre de copies intégrées et donc le niveau d'expression qui en résulte ne peuvent être contrôlés. De nombreux efforts sont donc mis en oeuvre pour optimiser ces systèmes de production et différentes voies d'amélioration sont explorées, qui concernent : les vecteurs d'expression et leurs modalités d'intégration, les cellules et leurs conditions de culture et l'amplification génique (14-26).

L'ensemble de ces efforts s'est surtout concentré sur les lignées utilisées pour la production à l'échelle industrielle soit, principalement, les lignées CHO (14, 25, 27-29) et NS0 (22, 30, 31) ou bien encore les lignées HEK293 (32, 33) ou BHK (34-36). Toutefois, la sélection d'un clone fortement producteur reste encore très souvent une étape critique qui nécessite le criblage d'un grand nombre de transfectants en raison du caractère aléatoire de l'intégration du vecteur d'expression dans le génome de la lignée d'expression. En effet, après intégration du vecteur dans le génome, le niveau d'expression de la protéine recombinante est fortement influencé par l'« effet de position », c'est à dire par l'environnement génétique du locus d'intégration (37, 38). Ainsi, l'insertion du vecteur d'expression dans une région transcriptionnellement inactive du génome résultera en une expression faible, voire nulle, tandis que l'intégration du vecteur dans une région transcriptionnellement active pourra entraîner une forte expression.

La majeure partie du génome se trouvant dans un état transcriptionnellement inactif, il est en général nécessaire de cribler un grand nombre de transfectants pour isoler un clone fortement producteur (22).

### Art antérieur

Afin de contrecarrer cet « effet de position », différentes stratégies sont explorées. L'une de ces approches consiste à supprimer l'effet de position par l'intermédiaire d'isolateurs ou de séquences LCR (Locus Control Region) (39-42). Dans ce cas, l'expression du transgène est uniquement dépendante du nombre de copies du vecteur ayant été intégrées et de l'efficacité des séquences exogènes dirigeant l'expression de la protéine recombinante.

Une autre démarche repose sur la suppression de l'effet de position par l'addition de séquences induisant un état de chromatine favorable à l'activité transcriptionnelle (UCOE) (43).

D'autres stratégies consistent, au contraire, à supprimer le caractère aléatoire de l'intégration en dirigeant l'insertion du vecteur d'expression au niveau d'un locus favorable à l'expression du transgène. Dans ce cas l'intégration peut être ciblée au niveau d'un gène connu (Hollis GF, US 6,750,041) ou bien de sites présentant une bonne activité transcriptionnelle (44, 45, Reff MR, brevet US 6,841,383). Grâce à cette démarche l'événement d'intégration est contrôlé et dirigé vers un locus garantissant l'expression du transgène à un niveau constant et, si possible, élevé. L'étape de transfection ne constitue donc plus un événement aléatoire mais devient un événement reproductible, avec pour conséquences, l'allègement des étapes de criblage et de caractérisation ainsi que la garantie d'une productivité comparable d'une transfection à l'autre.

L'intégration ciblée du vecteur d'expression peut être réalisée par recombinaison homologue (Reff MR, brevet US 6,841,383 ; Hollis GF, brevet US 6,750,041 ; 46, 47). Toutefois, si cette forme de recombinaison intervient naturellement avec une fréquence élevée dans la levure ou dans d'autres organismes fongiques, elle se révèle en fait beaucoup plus rare chez les eucaryotes supérieurs, ce qui constitue un obstacle important quant à son utilisation dans ce type d'organismes (47). Par exemple, dans les cellules de mammifères, le rapport de la fréquence de recombinaison homologue sur la fréquence de l'intégration aléatoire se situe entre 1/100 et 1/5000 (48). Une approche complémentaire destinée à améliorer l'efficacité de la recombinaison homologue consiste, par ailleurs, à provoquer l'apparition de coupures dans l'ADN en utilisant des méganucléases (par exemple I-Sce I)(49).

Une autre démarche consiste en l'utilisation de recombinases (47, 50), et notamment en l'utilisation des recombinases Cre (51, 52) ou Flp (53, 54). Le système de recombinaison CreloxP du bactériophage P1 a ainsi été adapté et utilisé pour cibler certains gènes présents dans les cellules eucaryotes (51, 52, Sauer BL, EP 0 220 009). L'intégration ciblée dans le génome de cellules CHO (Chinese hamster ovary) au moyen de la recombinase Cre a par exemple été décrite (44). Ce système de recombinaison est attractif en ce qu'il permet une expression reproductible en un même locus du génome. Toutefois, une telle approche permet uniquement de s'assurer de la reproductibilité des niveaux d'expression d'un gène rapporteur mais elle ne permet pas de garantir une forte expression du gène concerné.

Afin de pallier ce problème, des lignées cellulaires constituant des hôtes d'expression stables et possédant un site de recombinaison FRT dans une région du génome transcriptionnellement active ont été créées (lignées cellulaires Flp-In™, Invitrogen). L'intégration d'un vecteur d'expression, dans le site localisé au sein de la région transcriptionnellement active est obtenue par une recombinaison de type Flp-FRT, ce qui assure une forte transcription du gène d'intérêt. Toutefois les lignées Flp-in proposées (293, CHO, BHK, 3T3) ne sont pas toujours optimales quant à la production de certaines protéines recombinantes, comme les anticorps monoclonaux par exemple, en raison de leurs propriétés intrinsèques de modification post-traductionnelle. Par ailleurs, ces lignées cellulaires comportent également dans leur génome des séquences additionnelles permettant de sélectionner les cellules ayant correctement intégré les sites de recombinaison au sein d'une région fortement transcrite.

Il reste cependant préférable de pouvoir disposer de lignées cellulaires « hôtes » dépourvues de toute séquences actives (promoteur, activateurs ou gène de résistance aux antibiotiques) afin de ne pas restreindre l'utilisation de gènes de résistance ou de séquences régulatrices (promoteurs, activateurs, polyA) susceptibles d'être utilisés pour l'expression ultérieure de la protéine d'intérêt. Enfin, la re-expression éventuelle d'un gène rapporteur concomitante à l'expression de la protéine d'intérêt peut imposer des vérifications complexes, notamment dans le cadre de la production de protéines thérapeutiques, soumise à des normes réglementaires très strictes, notamment en ce qui concerne l'ingénierie moléculaire des lignées cellulaires produisant les protéines d'intérêt thérapeutique.

Plus récemment Kito et al (45) ont adapté l'approche Cre-loxP à la sélection de clones fortement producteurs dans les cellules CHO en utilisant le gène rapporteur codant la GFP, ce qui a permis d'obtenir des niveaux d'expression avoisinant 160 mg/l après amplification génique. Cependant, comme dans le cas des lignées Flp-In précédemment évoquées, cette stratégie ne permet pas de s'affranchir de la présence de multiples séquences actives dans la lignée cellulaire utilisée en tant qu'hôte pour réaliser l'expression.

La production de protéines thérapeutiques recombinantes étant soumise à des normes réglementaires très strictes au niveau de l'ingénierie moléculaire des lignées cellulaires produisant les protéines d'intérêt thérapeutique, la présence de séquences actives dans le génome de ces cellules complique donc l'élaboration du dossier réglementaire, et rallonge les phases de développement des médicaments ainsi produits.

Ainsi, la mise au point de nouvelles lignées cellulaires fortement productrices, stables dans le temps, permettant de produire de manière simplifiée tout type de protéines d'intérêt industrielle, et notamment des protéines thérapeutiques, reste par conséquent un enjeu majeur pour améliorer la production de protéines recombinantes à partir de cellules eucaryotes et, en particulier, à partir de cellules de mammifères.

Selon les différents objets faisant partie de la présente invention, il est fait référence à une protéine d'intérêt, qui puisse être exprimée dans la cellule, soit pour les besoins de la vérification de l'intégration des sites FRT, soit, dans la vision globale de l'invention, pour la production de telles protéines à l'échelle industrielle. Telle qu'elle est présentée dans la présente description, abrégé, et revendications, cette expression signifie que la lignée cellulaire produit des protéines qui peuvent ou non être présentes nativement dans la cellule, et les surexprime au-delà de leur taux d'expression native.

### Définitions

De manière générale, aussi bien dans la description, et abrégé, que dans les revendications, les termes suivants qui sont rencontrés ont les significations suivantes, sauf stipulation différente :
Par « **séquence d'acide nucléique** », on entend, au sens de la présente invention, un oligomère ou un polymère, simple ou double brin, de bases nucléotidiques lues à partir de l'extrémité 5' vers l'extrémité 3'. Le terme « séquence d'acide nucléique » peut se référer à une molécule d'ADN, d'ARN ou à une molécule hybride ARN/ADN, d'origine naturelle ou synthétique. Dans la notation nucléotidique utilisée dans la présente demande, sauf mention particulière, l'extrémité gauche d'une séquence nucléotidique simple brin est l'extrémité 5'.
Par « **molécule d'ADN** », on entend, au sens de la présente invention, tout oligomère ou polymère, simple ou double brin, constitué par un enchaînement de bases nucléotidiques, d'origine naturelle ou synthétique et comprenant, sans y être limité, un gène, un ensemble de gènes, un fragment de gène, un mélange de séquences codantes et non codantes, des séquences de régulation, ou la séquence complémentaire de celle correspondant à un ARN.
Par « **promoteur** » ou « **séquence promotrice** », on entend, au sens de la présente invention, une séquence d'acide nucléique, naturelle ou synthétique, située en amont du codon de démarrage de la traduction et impliquée dans la reconnaissance et la liaison de l'ARN polymérase. Cette séquence promotrice permet ainsi d'initier la transcription d'une région codante située en aval. De tels promoteurs sont connus par l'homme du métier et peuvent comprendre des promoteurs bactériens, viraux, eucaryotes, de levure ou de mammifère, la sélection du promoteur étant dépendante du système cellulaire hôte utilisé pour réaliser l'expression.
Par « **vecteur** », on entend, au sens de la présente invention, un véhicule de transit à base d'acides nucléiques, une molécule d'acide nucléique adaptée pour livrer de l'acide nucléique ou une molécule d'ADN capable de réplication autonome dans une cellule hôte, par exemple un plasmide, un cosmide, un phagemide, un génome (chromosome) viral, un génome (chromosome) phagique, et qui permet le clonage de molécules d'ADN. Selon l'hôte cellulaire considéré et la nature des séquences nucléiques constituant le vecteur, le vecteur peut être soit répliqué de manière stable par les cellules en tant que structure autonome, soit intégré au génome de l'hôte, soit maintenu dans le noyau ou le cytoplasme de l'hôte.
Par « **plasmide** », on entend, au sens de la présente invention, une molécule d'ADN circulaire autonome, éventuellement linéarisée, capable de réplication dans une cellule. Le terme plasmide comprend à la fois les plasmides dits « d'expression » et les plasmides dits « de non-expression ». Lorsque le plasmide est maintenu par une cellule hôte, il peut être soit répliqué de manière stable par les cellules en tant que structure autonome, soit être intégré au génome de l'hôte.
Par « **peptide** », « **polypeptide** » ou « **protéine** », on entend, au sens de la présente invention, une séquence primaire d'acides aminés liés par des liaisons peptidiques covalentes. En général, un peptide, plus court qu'une protéine, est constitué d'un faible nombre d'acides aminés, typiquement de 2 à 50. Le terme polypeptide peut recouvrir à la fois les peptides et les protéines. Un peptide, un polypeptide ou une protéine peuvent être d'origine synthétique, recombinante ou naturelle.
Par « **signal de terminaison de la transcription** », on entend, au sens de la présente invention, une séquence d'acide nucléique placée à la fin d'une région transcrite et qui provoque l'arrêt de la transcription de cette région par l'ARN polymérase. Des exemples de signaux de terminaison de la transcription utiles dans le cadre de la présente invention comprennent, sans y être limités, des séquences de polyadénylation, comme par exemple la séquence de polyadénylation « SV40 early polyadenylation signal », et la séquence de polyadénylation BGH.
Par « **séquence de polyadénylation** » ou « **polyA** », on entend, au sens de la présente invention, une séquence d'ADN qui provoque à la fois la terminaison de la transcription et la polyadénylation de l'ARN transcrit naissant. Une polyadénylation efficace des transcrits est généralement souhaitable dans la mesure où les transcrits dépourvus de queue polyA s'avèrent souvent instables et sont rapidement dégradés.
   Un clivage et une polyadénylation efficaces des ARN messagers de mammifères requièrent au moins deux éléments de signal: une séquence AAUAAA située 7 à 30 paires de bases en amont d'un site de processing, et des séquences riches en GU ou U situées en 3' du site de clivage.
Par « séquence de **polyadénylation présentant une faible efficacité** », on entend, au sens de la présente invention, une séquence de polyadénylation ne permettant pas une mise en oeuvre efficace de la terminaison de la transcription et de la polyadénylation des transcrits. Il en résulte une faible quantité de transcrits et/ou une instabilité importante des transcrits, qui sont, pour la plupart, dégradés trop rapidement pour permettre leur traduction. Plus particulièrement, on entend par « séquence de polyadénylation présentant une faible efficacité » toute séquence de polyadénylation permettant une mise en oeuvre de la terminaison de la transcription et de la polyadénylation des transcrits à un niveau inférieur ou égal à celle induite par la séquence de polyadénylation « SV40 early polyadenylation signal ». Toute séquence de polyadénylation permettant d'atteindre cet objectif peut être utilisée dans le cadre de l'invention. Plus particulièrement, ces séquences peuvent être des séquences de polyadénylation intègres présentant un signal de polyadénylation peu efficace de la terminaison de la transcription et de la polyadénylation des transcrits, comme par exemple le polyA SV40 early, ou le polyA de l'adénovirus L1 (71), ou bien des séquences de polyadénylation mutées ou délétées de manière à diminuer le niveau de mise en oeuvre de la terminaison de la transcription et de la polyadénylation des transcrits par rapport à la séquence non mutée ou non délétée. On peut citer à titre d'exemple de mutation et/ou de délétion d'une séquence de polyadénylation, permettant de diminuer l'efficacité du signal de polyadénylation par rapport à la séquence non mutée, l'augmentation de la distance séparant les éléments AATAAA et la région riche en GT (66), la délétion du polyA SV40 late d'un ou plusieurs nucléotides présents en amont de l'hexanucléotide AATAAA (67), la délétion de certains éléments situés entre le nucléotides situés de 13 à 48 nucléotides en amont de la séquence AATAAA (68), la délétion des séquences riches en GT en aval de la séquence AATAAA et la modification de l'espace situé entre la séquence AATAAA et la région riche en GT (69), ou la mutation ou la délétion des régions USE (upstream sequence elements) situées en amont de la séquence AATAAA (70), cette liste n'étant pas limitative.
Par **« isolé » ou « purifié** », on entend, au sens de la présente invention, toute modification réalisée par la main de l'homme à partir de l'état naturel. Par conséquent, tout objet préexistant dans la nature ayant été modifié ou extrait de son environnement naturel est dit « isolé » ou « purifié ». Un objet « isolé » peut correspondre à tout polynucléotide ou à tout(e) peptide/polypeptide/protéine séparé(e) des molécules coexistantes dans son environnement naturel, obtenu par clonage, par amplification et/ou par synthèse chimique. Par ailleurs, un polynucléotide ou un(e) peptide/protéine qui est introduit dans un organisme par transformation, manipulation génétique ou par toute autre méthode, est dit « isolé » même s'il préexistait dans cet organisme.
Par « **expression** », on entend, au sens de la présente invention, la transcription et/ou la traduction d'une séquence polynucléotidique particulière, placée sous le contrôle d'une séquence régulatrice telle qu'un promoteur, par exemple.
Par « **surexpression** », on entend, au sens de la présente invention, un niveau d'expression pour une séquence codante donnée qui est significativement plus élevé (par exemple deux fois, et de préférence 10 fois ou même une centaine de fois) par cellule que le niveau par cellule observé avec la séquence codante native correspondante dans une cellule non transfectée par la construction de l'invention.
Par « **anticorps** », on entend, au sens de la présente invention, une molécule d'immunoglobuline immunologiquement réactive à un antigène particulier, et qui inclut, selon les cas, les anticorps polyclonaux et/ou monoclonaux. Ce terme comprend aussi les formes génétiquement modifiées, telles que les anticorps chimériques (par exemple les anticorps de souris ou de lapin humanisés), ou les anticorps hétéroconjugués (par exemple les anticorps bispécifiques). Le terme « anticorps » comprend également les formes d'anticorps se liant aux antigènes, y compris les fragments d'anticorps ayant la capacité de se lier aux antigènes.
Par « **transfection »** ou « **transfecter** », on entend, au sens de la présente invention, un procédé par lequel des cellules incorporent un ADN exogène et intègrent cet ADN dans leur génome.
Par « **lignée cellulaire** », on entend, au sens de la présente invention, un ensemble de cellules provenant d'une même cellule mère et possédant les mêmes caractéristiques génétiques que cette cellule mère. Une lignée cellulaire se caractérise en outre par sa capacité à croître de façon stable in vitro pendant un grand nombre de générations.
Par « **zone de forte activité transcriptionnelle** », on entend, au sens de la présente invention, une région de l'ADN génomique ou chromosomique d'un organisme qui présente une organisation chromatinienne ou qui comporte des séquences régulatrices susceptibles d'augmenter de façon significative la fréquence de transcription des gènes présents dans ou aux abords de cette région. Le taux de transcription rencontré dans une telle zone d'activité est généralement supérieur au taux de transcription moyen classiquement observé dans le génome de cet organisme, et de préférence 2 fois supérieur, ou avantageusement 10 fois supérieur, voire 50 fois supérieur, et parfois même 100 fois supérieur. Par exemple, pour la lignée YB2/0 (ATCC CRL 1662), on considère qu'une zone de forte activité transcriptionnelle permet d'obtenir une valeur pcd (picogrammes de protéine/cellule/24 heures) supérieure ou égale à 5, et de préférence supérieure ou égale à 10.
Par « **zone de faible activité transcriptionnelle** », on entend, au sens de la présente invention, une région de l'ADN génomique ou chromosomique d'un organisme qui présente une organisation chromatinienne ou qui comporte des séquences régulatrices susceptibles de bloquer totalement ou de diminuer de façon significative la fréquence de transcription des gènes présents dans ou aux abords de cette région. Le taux de transcription rencontré dans une telle zone d'activité est généralement inférieur au taux de transcription moyen classiquement observé dans le génome de cet organisme, et de préférence 2 fois inférieur, ou avantageusement 10 fois inférieur, voire 50 fois inférieur, ou même 100 fois inférieur. Le taux de transcription rencontré dans une telle zone d'activité peut également se révéler si faible qu'il devient impossible de l'évaluer, et peut également être nul.
Par « **recombinase** » ou « **recombinase spécifique de site** », on entend, au sens de la présente invention, une enzyme dont l'action sur deux molécules d'acide nucléique consiste à provoquer une recombinaison entre lesdites deux molécules. La recombinaison est un procédé naturel bien connu qui conduit à la scission de deux molécules d'acides nucléiques ayant des séquences identiques ou essentiellement similaires (homologues), et qui mène à la reformation des deux molécules de sorte qu'une région de chaque molécule initialement présente devient liée à une région de l'autre molécule initialement présente. Deux types de réactions de recombinaison ont été identifiés. Le premier type, qui correspond à la recombinaison dite « classique » ou « homologue », va concerner n'importe quelle paire de molécules présentant des séquences nuléotidiques homologues susceptibles de servir de substrat à une recombinase « générale ». En revanche, dans le deuxième type de recombinaison, appelé « recombinaison spécifique de site », les molécules homologues doivent, pour servir de substrat à la recombinase, comporter une séquence nucléotidique particulière appelée « site de recombinaison spécifique ». Plusieurs systèmes de recombinaison spécifique de site sont décrits dans l'état de la technique, comme par exemple le système du bactériophage P1 de *E*. *coli.* En particulier, les séquences spécifiques et les recombinases utilisées peuvent appartenir à différentes classes structurales, et notamment à la famille de résolvase du transposon Tn3 ou à la famille de l'intégrase du bactériophage lambda. Parmi les recombinases appartenant à la famille du transposon Tn3, on peut citer notamment la résolvase du transposon Tn3 ou des transposons, Tn21 et Tn522 (Stark et al., 1992) ; l'invertase Gin du bactériophage mu ou encore les résolvases de plasmides, telle que celle du fragment par de RP4 (Abert et al., Mol. Microbiol. 12 (1994) 131). Parmi les recombinases appartenant à la famille de l'intégrase du bactériophage λ, on peut citer notamment l'intégrase des phages lambda (Landy et al., Science 197 (1977) 1147), P22 (Leong et al., J. Biol. Chem. 260 (1985) 4468), HP1 de Haemophilus influenzae (Hauser et al., J. Biol. Chem. 267 (1992) 6859), fintégrase Cre du phage P1, l'intégrase du plasmide pSAM2 (350341EPA EP 350 341) ou encore la FLP recombinase du plasmide 2µ et les recombinases XerC et XerD d'E coli.
Par « **site de reconnaissance à une recombinase** », on entend, au sens de la présente invention, une séquence d'acide nucléique susceptible de servir de substrat à une recombinase.
Par « **gène rapporteur** », on entend, au sens de la présente invention, un polynucléotide ayant une séquence encodant un produit de gène, de façon générale une enzyme, dont la présence peut être aisément détectée et/ou quantifiée lorsque la construction comprenant la séquence du gène rapporteur est introduite dans des cellules contenant tous les facteurs nécessaires à l'expression de ce gène. Des exemples de gènes rapporteurs utiles dans le cadre de la présente invention comprennent, sans y être limité, des protéines fluorescentes telles que la protéine maxFP-green et ses dérivés, la luciférase, la GFP (Green fluorescent protein) et ses dérivés, ou les RCFP (Reef Coral Fluorescent Proteins) ainsi que la beta-galactosidase encodée par le gène lacZ.
Par « **protéine d'intérêt** », on entend, au sens de la présente invention, tout peptide/polypeptide ou toute protéine susceptible de présenter un intérêt industriel, prophylactique ou thérapeutique. Les protéines d'intérêt susceptibles d'être exprimées dans les lignées cellulaires selon la présente invention peuvent être choisies parmi :
   - les protéines ayant une activité thérapeutique : c'est-à-dire, des protéines ayant un effet bénéfique physiologique reconnu sur le sujet humain ou animal dans une forme de maladie reconnue ou un dysfonctionnement pathologique dudit sujet animal ou humain, y compris dans des traitements prophylactiques; de telles protéines comprennent également des peptides, polypeptides, hormones, enzymes et semblables, et de préférence des polypeptides ayant une activité choisie parmi le groupe consistant en une activité dans les fonctions digestive, pancréatique, biliaire, antivirale, anti-inflammatoire, pulmonaire, antimicrobienne, hématologique, neurologique, cardiovasculaire, ophtalmologique, antigénique, cérébrale, anti-tumorale, immunostimulante, et immunomodulatrice; dans des modes d'exécution particulièrement préférés, la protéine ou le polypeptide, à activité thérapeutique, est choisi parmi le groupe consistant en les insulines ; une hormone de croissance, y compris une hormone de croissance humaine et une hormone de croissance bovine ; un facteur de relargage d'une hormone de croissance ; une hormone parathyroïdienne ; une hormone stimulant la thyroïde ; une hormone de stimulation des follicules ; une hormone de lutéïnisation; les interférons, tel qu'un interféron-alpha, -beta et -gamma ou, par exemple, un interféron 13 ; un facteur de croissance endothélial vasculaire (VEGF) ; des récepteurs pour des hormones ou des facteurs de croissance ; une intégrine ; une protéine A ou D ; des facteurs rhumatoïdes ; un facteur neurotrophique tel qu'un facteur neurotrophique dérivé de l'os (BDNF) ; une neurotrophine 3, 4, 5 ou 6 (NT-3, NT-4, NT-5 ou NT-6) ; un facteur de croissance de nerfs tel qu'un NGF-beta ; un facteur de croissance dérivé des plaquettes (PDGF) ; un facteur de croissance de fibroblastes tel qu'un FGF ou un bFGF ; un facteur de croissance épidermique (EGF) ; un facteur de croissance de transformation (TGF) tel qu'un TGF-alpha et un TGF-beta, comprenant un TGF-beta.1, un TGF-beta.2, un TGF-beta.3, un TGF-beta.4, ou un TGF-beta.5 ; un facteur de croissance ressemblant à l'insuline de type I et de type II (IGF-I et IGF-II), ou par exemple un des(1-3)-IGF-I (IGF-I du cerveau) ; un facteur de croissance des kératinocytes ; des protéines de liaison d'un facteur de croissance ressemblant à l'insuline ; des protéines CD telles qu'une CD-3, une CD-4, une CD-8, et une CD-19 ; une érythropoïétine ; des facteurs d'ostéoinduction ; des immunotoxines ; une protéine morphogénétique d'os (BMP) ; des facteurs de stimulation de colonies (CSFs), par exemple M-CSF, GM-CSF, et G-CSF ; des facteurs d'accélération du vieillissement ; les lipases gastriques, les lipases pancréatiques ou biliaires, les élastases, les anti-protéases telles que l'alpha-1 anti-trypsine ; les protéases ; les oxydases ; les phytases ; les chitinases ; les invertases ; les cellulases ; les xynalases ; les protéines structurantes telles que le collagène ; les transferrines telles que la lactoferrine ; les protéines dérivées du sang, telles que l'hémoglobine ou l'albumine humaine ; les cofacteurs sanguins, les facteurs de coagulation tels que le facteur VII, le facteur VIII, le facteur IX, le facteur X, le facteur tissulaire, le facteur von Willebrand ; les facteurs anti-coagulation tels qu'une protéine C ; le facteur natriurétique atrial ; la rénine ; la calcitonine ; le glucagon ; un surfactant pulmonaire ; un activateur du plasminogène, tel qu'une urokinase ou un activateur de plasminogène spécifique de tissus (t-PA) ; la thrombine ; la thrombopoïétine ; un facteur de croissance hématopoïétique, un facteur alpha ou beta de nécrose de tumeurs ; une enképhalinase ; une protéine d'inflammation des macrophages humains (MIP-1-alpha) ; une sérumalbumine telle que la sérumalbumine humaine ; la relaxine ; une DNase ; une cytokine ; les chimiokines telles que, par exemple un RANTES (regulated on activation normally T-cell expressed and secreted) ; des interleukines (ILs), par exemple IL-1 à IL-10 ; les antioxydants tels que la superoxide dismutase, les anticorps, les fragments d'anticorps et les antigènes. Lorsque le polypeptide ou protéine est choisi parmi les anticorps ou les fragments d'anticorps, ceux-ci peuvent comprendre des molécules d'immunoglobuline, la chaîne lourde d'immunoglobuline, des molécules d'immunoglobuline essentiellement complètes, et toutes parties d'une immunoglobuline comportant un paratope, y compris des fragments Fab, des fragments Fab', des fragments F(ab')₂ et des fragments Fv, la chaîne légère d'immunoglobuline, et des fragments Fv;
   - des protéines ou polypeptides à activité cosmétique qui, d'après les législation de nombreux pays, sont ceux qui exercent une activité uniquement au niveau de l'épiderme, c'est-à-dire que les molécules en question ne traversent pas jusqu'aux couches inférieures, ou autrement n'ont aucune action ou effet sur le derme ou les cellules basales. De telles protéines ou polypeptides sont connus de l'homme du métier en tant que tels, dont quelques exemples sont des céramides, des kératides, des agents hydratants, des agents antibactériens et semblables;
   - des protéines ou polypeptides à activité nutraceutique, c'est-à-dire des molécules qui sont identiques ou proches de celles que l'on trouve couramment dans le régime alimentaire humains ou animal, et qui peuvent se trouver en tout ou en partie dans l'alimentation ou une partie de cette dernière et qui ont un effet bénéfique sur la santé; en tant qu'exemples des types de molécules pouvant être concernées par cette partie de l'invention, on peut citer la PAL modifiée ou dérivée (phénylalanine ammonia lyase), les allergènes, par exemple de bouleau, de peuplier, et des Graminae, la superoxide dismutase (SOD), et semblables.
Par « **forte dose d'antibiotique** », on entend, au sens de la présente invention, une dose d'antibiotique supérieure ou égale à 1g/l, ou avantageusement supérieure ou égale à 2g/l, ou encore supérieure ou égale à 4g/l et de préférence à 8g/l.

Conformément à un aspect de la présente invention, il est prévu un procédé d'obtention d'une lignée cellulaire fortement productrice d'une protéine d'intérêt comportant au moins une cellule, comprenant les étapes suivantes :
- l'intégration dans une zone de forte activité transcriptionnelle du génome de ladite cellule d'une molécule d'acide nucléique comprenant (i) deux séquences en tandem correspondant chacune à un site de reconnaissance d'une recombinase, entre lesquelles se trouvent un gène rapporteur, un gène codant une protéine d'intérêt, un gène de sélection et un gène de résistance à un antibiotique dépourvu de séquence de polyadénylation, et (ii) une séquence signal de polyadénylation de faible efficacité située en aval du deuxième site de reconnaissance d'une recombinase ;
- La sélection des cellules fortement productrices ayant intégré une copie unique de la molécule d'acide nucléique ;
- L'excision de la molécule d'acide nucléique par l'action de la recombinase ; et,
- La sélection des cellules formant des lignées cellulaires qui ont excisé la molécule d'acide nucléique en ne conservant qu'un site unique et intègre de reconnaissance d'une recombinase.

Selon un autre mode particulièrement avantageux de la présente invention, la ou les cellules de la lignée cellulaire utilisée dans les procédés de la présente invention est une cellule de mammifère ou une cellule aviaire. La cellule de départ, c'est- à-dire avant modification, est ainsi choisie parmi le groupe consistant en : les lignées de myélomes de rat, notamment YB2/0 (ATCC CRL-1662) et IR983F, de myélome humain comme Namalwa ou toute autre cellule d'origine humaine comme PERC6, les lignées CHO, notamment CHO-K, CHO-LeclO, CHO-Lecl, CHO Pro-5, CHO dhfr- , CHO Lecl3, ou d'autres lignées choisies parmi Wil-2, Jurkat, Vero, Molt-4, COS-7, 293-HEK, BHK, K6H6, NSO, SP2/0-Ag 14, P3X63Ag8.653, et Ebx.

Selon la présente invention, l'intégration du site unique de reconnaissance d'une recombinase est effectuée par une série d'étapes comprenant au moins :
- l'intégration de deux séquences en tandem d'acide nucléique codant chacune un site de reconnaissance d'une recombinase ;
- l'intégration d'une séquence d'acide nucléique, codant un gène rapporteur, entre les deux séquences codant chacune un site de reconnaissance d'une recombinase ;
- l'intégration d'une séquence d'acide nucléique codant une protéine d'intérêt entre les 2 séquences codant chacune un site de reconnaissance d'une recombinase ; et
- l'intégration d'une séquence d'acide nucléique, codant un marqueur de sélection, et un gène de résistance à un antibiotique dépourvu de séquence de polyadénylation entre les 2 séquences codant chacune un site de reconnaissance d'une recombinase, et
- L'intégration d'une séquence signal de polyadénylation de faible efficacité située en aval du deuxième site de reconnaissance d'une recombinase.

Dans ce cas, la séquence de reconnaissance de la recombinase peut être la séquence loxP et/ou FRT. La protéine d'intérêt peut être par exemple un anticorps ou un fragment d'anticorps.

Il est également préféré de ne sélectionner que les cellules fortement productrices ayant intégré une seule copie de l'ensemble des séquences mentionnées précédemment. De manière encore plus préférée, seules les cellules ayant une valeur pcd (pg de protéine/cellule/24 heures) égale ou supérieure à 5, et de préférence égale ou supérieure à 10, ou encore à 20, à 30, à 50, à 80 ou à 100, sont sélectionnées.

Par la suite, on procède à l'excision de l'ensemble des séquences mentionnées précédemment par action d'une recombinase sur la cellule. Dans ce cas, l'action d'une recombinase peut être obtenue par co-expression dans la cellule de ladite recombinase au moyen d'un vecteur comportant une séquence d'acide nucléique codant ladite recombinase.

La série d'étapes mentionnées précédemment comprend en outre la sélection des cellules ayant excisé l'ensemble des séquences d'acide nucléique mentionnées précédemment, et possédant un site de reconnaissance unique et intègre de la recombinase.

En outre, la série d'étapes peut, de manière encore plus préférée, comprendre l'intégration d'une séquence d'acide nucléique , codant la thymidine kinase du virus Herpès simplex de type I (HSV1-TK). Dans ce cas, la sélection des cellules peut être effectuée par l'ajout de ganciclovir dans le milieu de culture. En effet, cette sélection permet de s'assurer que seules les cellules ayant intégré la séquence d'acide nucléique codant ladite HSV1-TK résisteront à la culture avec le ganciclovir présent dans le milieu.

La série d'étapes mentionnées précédemment comprend, en outre, et ce de manière particulièrement préférée, une transfection de la lignée cellulaire sélectionnée avec un vecteur d'expression comprenant une séquence d'acides nucléiques codant une protéine ou polypeptide d'intérêt et une séquence d'acide nucléique codant un site de reconnaissance de recombinase directement en aval d'une séquence d'acide nucléique codant un marqueur de sélection, de préférence de résistance à un antibiotique, dépourvu de séquence de polyadénylation. Dans ce cas, on insère ledit vecteur d'expression au niveau du site unique de reconnaissance de recombinase, grâce à l'action combinée de ladite recombinase, qui est exprimée ou apportée en même temps que la transfection. La sélection des cellules contenant le vecteur d'expression intégré au site cible peut être avantageusement effectuée en testant l'expression de la protéine ou le polypeptide d'intérêt. Avantageusement les cellules pourront être également sélectionnées pour leur résistance en présence d'une forte concentration d'antibiotique, en particulier leur résistance à une dose d'antibiotique supérieure ou égale à 1g/l, ou à 2g/l, ou de préférence à 4g/l, ou à 8g/l.

De manière particulièrement préférée, ladite protéine ou ledit polypeptide d'intérêt est une protéine ou polypeptide thérapeutique, notamment choisi parmi le groupe présentant une activité dans les fonctions digestive, pancréatique, biliaire, antivirale, anti-inflammatoire, pulmonaire, antimicrobiale, hématologique, neurologique, cardiovasculaire, ophthalmologique, antigénique, cérébrale, anti-tumorale, immunostimulante, et immunomodulatrice.

Les étapes de procédé décrites ci-dessus, à leurs différents stades, permettent d'obtenir une ou plusieurs lignées cellulaires présentant des caractéristiques particulièrement avantageuses. Par conséquent, un autre objet de la présente invention est une lignée cellulaire comprenant, intégré de manière stable dans son génome, un site unique de reconnaissance d'une recombinase dans une zone de forte activité transcriptionnelle du génome de ladite cellule et, directement en aval dudit site unique de reconnaissance d'une recombinase, une séquence d'acide nucléique codant un signal de polyadénylation de faible efficacité. Cette séquence d'acide nucléique codant un signal de terminaison de la transcription ou de polyadénylation est une séquence codant tout ou partie d'un signal de poyadenylation de faible efficacité tel que le signal de polyadénylation SV40 précoce ou tout autre signal de polyadénylation modifié de façon à en altérer l'efficacité, tel que défini plus haut. Comme cela a été dit plus haut, on préfère que la lignée cellulaire soit constituée de cellules de mammifère.

La lignée cellulaire telle que décrite ici peut donc avantageusement comprendre en outre :
- deux séquences d'acide nucléique codant chacune un site de reconnaissance d'une recombinase ;
- au moins une séquence d'acide nucléique codant une protéine d'intérêt entre les 2 séquences codant chacune un site de reconnaissance d'une recombinase ; et
- au moins une séquence d'acide nucléique, codant un marqueur de sélection, de préférence un gène de résistance à un antibiotique, dépourvue de séquence de polyadénylation, située entre les deux séquences codant chacune un site de reconnaissance d'une recombinase, cette séquence codant pour un marqueur de sélection étant située directement en amont du site de reconnaissance d'une recombinase, lui même situé directement en amont de la séquence de polyadénylation faible précédemment décrite.

De préférence, et comme cela a été dit précédemment, l'ensemble des séquences mentionnées ci-dessus sont intégrées ensemble en une seule copie dans le génome des cellules de la lignée cellulaire. De manière plus préférée encore, chaque cellule de la lignée cellulaire présente une valeur pcd égale ou supérieure à 5, ou de manière préférée supérieure ou égale à 10.

La lignée cellulaire peut également, et ce de manière très préférée, surexprimer une protéine ou polypeptide d'intérêt choisi parmi le groupe des protéines ou polypeptides d'intérêt présentant une activité dans les fonctions digestive, pancréatique, biliaire, antivirale, anti-inflammatoire, pulmonaire, antimicrobiale, hématologique, neurologique, cardiovasculaire, ophtalmologique, antigénique, cérébrale, anti-tumorale, immunostimulante, et immunomodulatrice.

Selon un autre objet de la présente invention, la lignée cellulaire est celle identifiée par la référence YGM-1/10G10, et déposée sous le numéro de dépôt CNCM I-3704 (lignée déposée à la CNCM, Collection Nationale de Cultures de Microorganismes, Institut Pasteur, 25 rue du Docteur Roux, 75724 Paris Cedex 15, le 18 décembre 2006).

Selon encore un autre objet de la présente invention, la lignée cellulaire est la lignée identifiée par la référence YGM-2/3G5, et déposée sous le numéro de dépôt CNCM 1-3885 ((lignée déposée à la CNCM, Collection Nationale de Cultures de Microorganismes, Institut Pasteur, 25 rue du Docteur Roux, 75724 Paris Cedex 15, le 19 décembre 2007).

La demande de brevet décrit une molécule d'acide nucléique isolée comprenant un fragment d'acide nucléique identifié par le numéro SEQ ID NO :1. Cette molécule représente ou indique un site de haute activité transcriptionnelle, et peut donc servir pour l'intégration des autres séquences mentionnées ci-dessus. Il va de soi que la séquence SEQ ID NO :1 pourrait également servir, directement, ou de manière complémentaire, ou par hybridation, à créer un site de haute activité transcriptionnelle dans le génome d'une cellule appropriée. Ceci pourrait être effectué en passant par un vecteur comportant une séquence d'acide nucléique telle qu'identifiée dans le listage de séquences par le numéro SEQ ID NO : 1.

Selon encore un autre objet, particulièrement préféré, de la présente invention, on prévoit un procédé de production d'au moins une protéine ou polypeptide d'intérêt, caractérisé en ce qu'on met en culture une lignée cellulaire telle que décrite ci-dessus, de manière à exprimer ladite protéine ou polypeptide d'intérêt, suivi d'au moins une étape de récupération de ladite protéine d'intérêt. Les lignées cellulaires telles que celles décrites et identifiées par leur numéro de dépôt donné ci-dessus conviennent parfaitement à ce type de procédé. Dans ce cas, la protéine ou polypeptide d'intérêt est choisi de préférence parmi le groupe des protéines ou polypeptides d'intérêt présentant une activité dans les fonctions digestive, pancréatique, biliaire, antivirale, anti-inflammatoire, pulmonaire, antimicrobiale, hématologique, neurologique, cardiovasculaire, ophthalmologique, antigénique, cérébrale, anti-tumorale, immunostimulante, et immunomodulatrice. De manière encore plus préférée, la protéine ou polypeptide d'intérêt est un anticorps ou fragment d'anticorps.

L'invention a également pour objet une lignée cellulaire obtenue selon le procédé de l'invention.

### FIGURES

**Figure 1** : vecteur de ciblage pTV1
**Figure 2** **:** vecteur d'expression de la recombinase pFlpe
**Figure 3** **:** vecteur d'expression pT125FRT
**Figure 4** **:** vecteur T125-IG24
**Figure 5** **:** schéma de délétion du vecteur de ciblage par la recombinase Flp
**Figure 6** : schéma de réintégration du vecteur d'expression par la recombinase Flp

### Description détaillée

Un premier objet de l'invention se rapporte à un procédé permettant d'insérer une molécule d'ADN d'intérêt au sein d'un site cible dans le génome d'une cellule de mammifère. Ce procédé comporte une première étape consistant en l'obtention d'une lignée cellulaire dans le génome de laquelle un site unique de reconnaissance d'une recombinase est intégré au sein d'une zone de forte activité transcriptionnelle. Cette première étape comprend les étapes suivantes :
1) L'intégration dans une cellule de mammifère d'un premier acide nucléique, appelé « vecteur de ciblage », contenant (i) deux séquences en tandem correspondant à des sites de reconnaissance d'une recombinase, entre lesquelles se trouvent un gène rapporteur, un gène codant une protéine similaire aux protéines d'intérêt, un gène de sélection permettant de réaliser une amplification génique et un gène de résistance à un antibiotique mais dépourvu de séquence de polyadénylation ; et (ii) un site de polyadénylation de faible efficacité situé en aval du deuxième site de reconnaissance d'une recombinase, c'est à dire localisé en 5' du gène de résistance à un antibiotique ;
2) la sélection des cellules « fortement productrices » ayant intégré une copie unique du vecteur de ciblage ;
3) l'excision du vecteur de ciblage par l'action de la recombinase ; et
4) la sélection des cellules formant des lignées cellulaires qui ont excisé le vecteur de ciblage en ne conservant qu'un site unique et intègre de reconnaissance d'une recombinase.

Grâce au procédé de l'invention, les cellules formant des lignées cellulaires sélectionnées à l'étape 4) possèdent toutes un site unique de reconnaissance d'une recombinase intégré dans une zone de forte activité transcriptionnelle de leur génome.

Une des caractéristiques de la cellule de mammifère mise en oeuvre dans l'étape 1) du procédé, est qu'elle ne comporte pas, avant sa modification par le procédé de l'invention, de séquence identique ou similaire à celle du site de reconnaissance d'une recombinase intégré par la mise en oeuvre du procédé de l'invention. Par conséquent, le site de reconnaissance d'une recombinase intégré dans le génome des cellules de la lignée cellulaire de l'invention est unique, puisque aucune autre séquence identique n'est rencontrée dans le génome des cellules de cette lignée.

L'intégration d'un premier acide nucléique dans une cellule de mammifère, telle qu'elle est mise en oeuvre dans l'étape 1) du procédé, peut être réalisée par tous les moyens connus de l'homme du métier. On peut citer à titre d'exemple la technique de précipitation au phosphate de calcium (CaPO₄), par laquelle l'ADN précipité va être « intégré » par les cellules via un processus de phagocytose ; ou bien la technique de lipofection, qui consiste à enrober l'ADN à insérer dans des vésicules de lipides pour permettre la fusion avec la membrane cellulaire de la cellule hôte. Une autre technique susceptible d'être mise en oeuvre est la technique d'électroporation, qui permet à la cellule, via un choc électrique, d'intégrer l'ADN d'intérêt. Une autre technique peut être celle de la microinjection pronucléaire.

Le vecteur de ciblage utilisé dans la première étape du procédé de l'invention consiste en une molécule d'acide nucléique contenant deux sites de reconnaissance de recombinase. Ces sites sont identiques entre eux, et sont identiques au site unique présent dans la lignée cellulaire obtenue par la mise en oeuvre du procédé de l'invention. Ces deux sites délimitent un fragment d'ADN qui contient un gène rapporteur, un gène codant une protéine similaire aux protéines d'intérêt, un gène de résistance à un antibiotique et un gène de sélection permettant une amplification génique. Le vecteur de ciblage comporte en outre toutes les séquences qui s'avèrent nécessaires pour permettre l'expression des gènes placés entre les deux sites de reconnaissance d'une recombinase. Parmi ces séquences nécessaires, on peut citer, de manière non limitative, les séquences promotrices, activatrices, et de polyadénylation par exemple.

L'intégration du vecteur de ciblage se produit de manière aléatoire dans le génome de la cellule transfectée. Le vecteur de ciblage pourra donc s'intégrer dans des zones dépourvues d'activité transcriptionnelle, ou dans des zones possédant une activité transcriptionnelle faible, modérée ou élevée. Les lignées cellulaires ayant intégré le vecteur de ciblage dans une zone de forte activité transcriptionnelle sont sélectionnées en se fondant sur l'expression ou la surexpression d'un gène rapporteur.

L'expression de ce gène rapporteur, aisément visualisable et analysable, constitue un témoin capable de refléter l'importance de l'activité transcriptionnelle de l'environnement dans lequel ce gène rapporteur est placé : si l'activité transcriptionnelle dans la zone d'insertion est faible, l'activité du gène rapporteur sera faible. A *contrario,* si l'activité transcriptionnelle dans la zone d'insertion est forte, l'activité du gène rapporteur sera élevée.

Le gène codant pour une protéine similaire aux protéines d'intérêt, situé en aval du gène rapporteur, permet de déterminer la capacité de la cellule contenant les transgènes à sécréter les protéines, ce qui ne peut pas être déterminé avec une protéine fluorescente, dont l'expression est intracellulaire. Toute protéine susceptible d'être facilement détectable peut convenir pour cette application. Parmi les protéines utilisables dans le cadre de la présente invention, on peut par exemple citer, de façon non limitative, le produit des gènes d'immunoglobuline, les facteurs de croissance, les interleukines, les facteurs de stimulation, les kinases, les facteurs de coagulation, l'alpha-antitrypsine, et l'albumine.

Le gène de résistance à un antibiotique permet la sélection des cellules transformées comportant le vecteur de ciblage. La sélection est réalisée en plaçant les cellules transformées en contact avec l'antibiotique correspondant puisque seules les cellules ayant intégré le vecteur de ciblage ont la possibilité de survivre. Le gène de résistance à un antibiotique est suivi d'une séquence de polyadénylation (ou « polyA »), qui joue un rôle important dans la stabilisation des ARNm correspondants (56-63). Toute séquence de polyadénylation connue de l'homme du métier et permettant l'expression du gène de résistance à un antibiotique peut être utilisée, mais on utilise préférentiellement une séquence polyA de faible efficacité.

Avantageusement, cette séquence de polyadénylation est une séquence de polyadénylation faible, c'est-à-dire une séquence de polyadénylation présentant une faible efficacité. Par conséquent, si l'intégration du vecteur de ciblage se produit dans une zone de faible activité transcriptionnelle, le gène de résistance à un antibiotique ne sera pas exprimé de façon suffisante pour permettre la survie de la cellule exposée à l'antibiotique. Au contraire, si la zone d'intégration possède une forte activité transcriptionnelle, le caractère « faible » de la séquence de polyadénylation n'empêchera pas le gène de résistance de s'exprimer, et rendra les cellules correspondantes résistantes à l'antibiotique. L'utilisation d'une telle séquence de polyadénylation faible, notamment lorsque la pression de sélection est forte (à de fortes doses d'antibiotique), permet de diminuer sensiblement le nombre de clones à cribler et contribue à l'identification de clones susceptibles de constituer de forts « producteurs » de protéines d'intérêt.

A titre d'exemple de séquence de polyadénylation faible, on peut citer la séquence de polyadénylation précoce de SV40, « SV40 early polyadenylation signal », utilisée dans certains vecteurs d'expression commerciaux (64-65).

Avantageusement les cellules pourront être également sélectionnées pour leur résistance en présence d'une forte concentration d'antibiotique. La combinaison de ces deux outils de sélection, à savoir l'utilisation d'une séquence de polyadénylation de faible efficacité, et celle d'une forte concentration d'antibiotique dans le milieu, fait de ce mode de sélection un moyen particulièrement avantageux pour sélectionner les cellules dans lesquelles le transgène s'est intégré à l'endroit voulu, c'est-à-dire au niveau du site de reconnaissance de la recombinase, sans sélectionner les cellules dans lesquelles le site de reconnaissance de la recombinase s'est intégré dans une zone induisant une activité transcriptionnelle plus faible. En effet, le fait que la séquence de polyadénylation ne présente qu'une faible efficacité a pour effet que le gène de résistance à l'antibiotique situé directement en amont de ce site n'est que faiblement exprimé. Ainsi, si la concentration en antibiotique ajoutée dans le milieu est élevée (par exemple supérieure ou égale à 1g/l, ou encore supérieure ou égale à 2g/l, ou à 4g/l, ou supérieure ou égale à 8g/l), seules les cellules ayant intégré le transgène dans un zone permettant une expression suffisamment forte du gène de résistance à l'antibiotique, et ce malgré la faible efficacité de la séquence de polyadénayltion, survivent. Ce mode de sélection mettant en oeuvre à la fois la faible efficacité de la séquence de polyadénylation et l'ajout d'une forte concentration en antibiotique dans le milieu de culture des cellules, a plusieurs avantages : il permet de cribler moins de clones, puisque les clones qui auront intégré le transgène ailleurs dans le génome qu'au niveau du site de reconnaissance à la recombinase meurent sous l'effet de la forte concentration en antibiotique présente dans le milieu. De plus les clones sélectionnés présentent de plus grandes capacités de production par rapport à un procédé de sélection effectué avec les procédés de l'art antérieur.

Avantageusement, le vecteur de ciblage est linéarisé par une enzyme de restriction préalablement à la transfection dans les cellules retenues. Afin de prévenir l'assemblage de plusieurs copies de vecteurs de ciblage avant l'étape d'intégration dans l'ADN génomique de la cellule, l'enzyme de restriction utilisée est choisie pour sa capacité à produire des extrémités franches. Par ailleurs, seules de faibles quantités de vecteur de ciblage sont utilisés pour réaliser la transfection. Ces précisions expérimentales permettent de limiter sensiblement le nombre de copies de vecteurs qui seront intégrées dans le génome de la cellule. En effet, il s'avère nécessaire, pour le bon déroulement de l'étape d'excision, que le vecteur de ciblage ne soit intégré qu'en copie unique au sein du génome de la cellule transfectée, de sorte que l'excision ultérieure ne pourra se produire qu'entre les deux sites de reconnaissance de la recombinase. Similairement, la bonne mise en oeuvre du procédé de l'invention ne pourra être atteinte qu'à la condition qu'une seule zone de forte activité transcriptionnelle aura été ciblée.

Le vecteur de ciblage comporte par ailleurs une séquence de polyadénylation située en aval du deuxième site de reconnaissance d'une recombinase. Cette séquence de polyadénylation est destinée à être utilisée lors de l'intégration ultérieure d'un nouveau vecteur comportant le gène d'une protéine d'intérêt. Ainsi qu'il sera détaillé plus loin, cette séquence de polyadénylation permettra de sélectionner les cellules ayant intégré le deuxième vecteur au niveau du site de reconnaissance d'une recombinase. Avantageusement, cette séquence de polyadénylation présente une faible efficacité et peut être considérée comme une « séquence de polyadénylation faible ». Il s'agit par exemple de la séquence de polyadénylation précoce de SV40, « SV40 early polyadenylation signal ».

Avantageusement, le vecteur de ciblage comporte un gène permettant de procéder à un mécanisme d'amplification génique. Ce gène peut, par exemple, être le gène de la dihydrofolate réductase (ou DHFR) ou de la glutamine synthetase (GS), des enzymes métaboliques essentielles à la survie cellulaire. Lorsque les cellules transfectées ayant intégré le vecteur de ciblage sont mises en culture en présence de doses croissantes d'un inhibiteur spécifique d'une des enzymes précitées (par exemple le méthotrexate pour la DHFR et la méthionine sulfoximine pour la GS), seuls les clones ayant multiplié leur nombre de copies du vecteur et ayant ainsi augmenté l'expression de l'enzyme DHFR ou GS pourront survivre (23, 30). Les taux d'amplification génique obtenus sont variables et dépendent essentiellement de la région du génome dans laquelle le vecteur s'est intégré. La zone d'intégration du vecteur de ciblage est donc sélectionnée en fonction de sa « capacité d'amplification », afin de s'assurer de la possibilité ultérieure d'amplifier l'expression du transgène d'intérêt.

Par ailleurs, dans un autre mode de réalisation particulier de l'invention, le vecteur de ciblage comporte, entre les deux sites de reconnaissance d'une recombinase, le gène de la thymidine kinase du virus Herpès simplex de type I (HSV1-TK). La sélection des cellules réalisée à l'étape 4) comprend alors l'ajout de ganciclovir dans le milieu de culture : les cellules n'ayant pas excisé le vecteur de ciblage seront tuées en présence de ganciclovir, alors que celles dont le vecteur de ciblage aura été excisé survivront. Il convient de noter que tout gène suicide utilisant une « prodrogue » pour devenir toxique, autre que HSV1-TK, peut être employé à cette étape. On peut citer, par exemple, les gènes CodA ou Fcy avec la prodrogue 5-Fluorocytosine (5-FC), cette liste n'étant pas limitative.

Le site de reconnaissance d'une recombinase utilisée dans le cadre de la présente invention peut correspondre à tout site connu de l'homme du métier. On peut citer, à titre d'exemple, le site loxP ou le site FRT. Dans un mode de réalisation de l'invention, les sites loxP et FRT sont utilisés simultanément. A titre d'exemple, ce mode de réalisation peut être utilisé pour insérer un vecteur d'expression, grâce à FRT, puis pour enlever le gène de sélection de ce vecteur après son intégration dans le génome, en utilisant loxP.

Le gène rapporteur codant pour une protéine similaire à une protéine d'intérêt peut correspondre à tout gène codant pour une protéine sécrétée similaire ou identique à une protéine d'intérêt thérapeutique ou industriel. On peut citer à titre d'exemple les gènes codant pour les anticorps, les facteurs de croissance, les interleukines, les facteurs de stimulation, les kinases, les facteurs de coagulation, l'alpha-1antitrypsine ou l'albumine, cette liste n'étant pas limitative.

L'expression additionnelle d'une protéine similaire à une protéine d'intérêt en plus de la protéine codée par le gène rapporteur permet, dès l'étape 2), de sélectionner les cellules non seulement en fonction du taux d'expression du gène rapporteur, mais également en fonction des capacités sécrétrices de ces cellules.

Avantageusement, la protéine d'intérêt est un anticorps. Dans ce cas, il s'avère particulièrement avantageux de placer dans le vecteur les gènes codant pour la chaîne lourde et pour la chaîne légère dudit anticorps. Par ailleurs, si la protéine d'intérêt est un anticorps, il convient, lors de l'étape 2), de sélectionner les cellules capables de produire des anticorps en fonction du taux ou de la forme de glycosylation recherchés.

Avantageusement, la cellule de mammifère de départ utilisée pour la mise en oeuvre du procédé de l'invention est choisie parmi : les lignées de myélomes de rat, notamment YB2/0 (ATCC CRL-1662, ref 59) et IR983F, les lignées de myélomes humains comme Namalwa ou toute autre cellule d'origine humaine comme PERC6, des lignées d'expression comme NS0, SP2/0, BHK, 293-HEK ou CHO, notamment CHO-K, CHO-Lec10, CHO-Lec1, CHO Pro-5, CHO dhfr-, CHO Lec13, des lignées aviaires comme EBx *(= lignée Vivalis),* des lignés mammaires ou encore d'autres lignées telles que Wil-2, Jurkat, Vero, Molt-4, COS-7, K6H6 et P3X63Ag8.653.

De manière particulièrement avantageuse, on choisit la lignée YB2/0.

Lors de l'étape 2) de sélection des cellules fortement productrices, on sélectionne les cellules dont le taux de production de la protéine d'intérêt est supérieur à 5 pcd (pg de protéine/cellule/24 heures). Avantageusement, le taux de production des cellules d'intérêt est supérieur à 10 pcd. De manière particulièrement avantageuse, le taux de production des cellules d'intérêt est supérieur à 15 pcd, et plus particulièrement à 20 pcd. Avantageusement, ce taux de production est compris entre 5 et 50 pcd, ou plus particulièrement entre 10 et 30 pcd.

L'estimation du nombre de copies intégrées dans la cellule de mammifère de départ peut être réalisée par toute technique connue de l'homme du métier. On peut citer à titre d'exemple la technique de PCR (Polymerisation Chain Reaction) quantitative.

Lors de l'étape d'excision du vecteur de ciblage (étape 3), la recombinase génère un évènement de recombinaison au niveau de la paire de sites de reconnaissance d'une recombinase, et provoque l'excision du vecteur de ciblage hors du génome cellulaire, tandis qu'un site de reconnaissance d'une recombinase demeure dans le génome. Le mécanisme d'excision mis en oeuvre dans le procédé de l'invention conduit à l'élimination des séquences nucléotidiques comprises entre les deux sites de reconnaissance d'une recombinase du vecteur de ciblage intégré. Le site de reconnaissance demeurant dans la zone de forte activité transcriptionnelle du génome conserve, pour sa part, la même orientation que les sites de reconnaissance initialement présents dans le vecteur de ciblage intégré. Par ailleurs, une séquence de polyadénylation demeure également dans la cellule de mammifère après l'excision du vecteur de ciblage, et se situe en aval du site restant de reconnaissance d'une recombinase. L'intégrité du site de reconnaissance de la recombinase demeurant dans les cellules pourra être contrôlée par toute technique connue de l'homme du métier. On peut citer à titre d'exemple les techniques de PCR (Polymerisation Chain Reaction) suivie d'un séquençage de l'ADN amplifié.

A titre d'exemple, la recombinase peut être exprimée dans la cellule par transfection transitoire d'un vecteur codant pour cette recombinase, avec lequel la cellule aurait été transfectée à l'issue de l'étape 2).

Il convient de noter que si le site de reconnaissance d'une recombinase est le site loxP, la recombinase à utiliser est Cre. Au contraire, si le site de reconnaissance est FRT, la recombinase à utiliser est flp. Avantageusement, dans le cadre de la présente invention, la recombinase utilisée est Flpe, une recombinase dérivée de Flp, qui possède une activité supérieure à Flp dans les conditions de culture appliquées pour des cellules de mammifère (55).

L'étape d'excision permet donc d'éliminer toutes les séquences actives susceptibles d'influencer l'expression ultérieure de futures protéines d'intérêt qui pourraient être insérées dans la zone de forte activité transcriptionnelle, par l'intermédiaire de la séquence de recombinaison FRT persistante dans le génome des cellules transfectées. La lignée cellulaire obtenue après l'excision du vecteur de ciblage ne conserve donc plus aucun élément actif d'origine transgénique.

Les lignées cellulaires produites dans le cadre de la première étape du procédé de l'invention, qui comprennent un site unique de reconnaissance d'une recombinase intégré dans une zone de forte activité transcriptionnelle de leur génome (« lignées cellulaires fortement productrices »), peuvent être utilisées pour la production de n'importe quelle protéine d'intérêt. La production d'une telle protéine pourra être réalisée en ciblant ultérieurement le site unique de recombinaison avec un vecteur comportant les séquences nécessaires à la transcription d'une protéine d'intérêt portées par ce vecteur. Une telle utilisation de la lignée cellulaire fortement productrice de l'invention sera explicitée plus loin.

Un autre objet de l'invention concerne un procédé tel que défini précédemment et comportant une autre étape (« étape de réintégration ») visant à insérer une molécule d'ADN d'intérêt dans une lignée cellulaire obtenue par la mise en oeuvre de la première étape du procédé de l'invention. Cette deuxième étape comprend les étapes suivantes :
5) la transfection d'une lignée cellulaire obtenue à l'issue de l'étape 4) avec un vecteur d'expression comprenant un gène codant pour une protéine d'intérêt, un gène permettant une amplification génique et un gène de résistance à un antibiotique, dépourvu de séquence de polyadénylation, celui-ci étant localisé directement en amont d'un site de reconnaissance d'une recombinase;
6) l'insertion dudit vecteur d'expression au niveau du site unique de reconnaissance d'une recombinase, grâce à l'action de ladite recombinase ;
7) la sélection des cellules contenant le vecteur d'expression intégré au site cible en testant l'expression de la protéine d'intérêt. Avantageusement, la sélection sera réalisée en présence d'une forte dose d'antibiotique afin de défavoriser les intégrations aléatoires.

Le vecteur d'expression utilisé au cours de l'étape de réintégration du procédé comporte un gène codant pour une protéine d'intérêt, un gène permettant une amplification génique ainsi que toutes les séquences nécessaires pour permettre l'expression des séquences codantes présentes dans ce vecteur, c'est-à-dire des promoteurs, des activateurs (ou enhancers) et des séquences de polyadénylation. Ce vecteur d'expression comporte aussi un gène de résistance à un antibiotique avec son promoteur, dépourvu de séquence de polyadénylation, localisé directement en amont d'un site de reconnaissance d'une recombinase.

Le gène de résistance à un antibiotique porté par ledit vecteur d'expression est, quant à lui, dépourvu de séquence de polyadénylation. Le gène de résistance à un antibiotique doit donc impérativement être situé à l'extrémité du vecteur afin de se placer directement en amont de la séquence de polyadénylation qui est demeurée dans le génome de la cellule après l'excision du vecteur de ciblage. Optionnellement, le gène de résistance à un antibiotique est le même que celui porté par le vecteur de ciblage. On peut alors réaliser l'étape de sélection des cellules en employant la même dose d'antibiotique que celle utilisée pour sélectionner les cellules fortement productrices à l'étape 2).

L'étape de sélection s'effectue donc en plaçant les cellules en contact avec l'antibiotique correspondant au gène de résistance.

Sous l'action de la recombinase, le vecteur d'expression est intégré dans le génome des cellules au niveau du site de recombinaison unique qui persiste à l'issue de l'étape 4. Pour ce faire, le site de reconnaissance d'une recombinase présente sur le vecteur d'expression est identique à celui qui a été intégré dans le génome de la cellule au cours de la première partie du procédé.

Comme décrit précédemment, si le site de reconnaissance d'une recombinase est loxP, la recombinase utilisée sera Cre. Si le site de reconnaissance d'une recombinase est FRT, la recombinase utilisée sera Flp. Par ailleurs, tout moyen permettant l'action de la recombinase dans la cellule peut être mise en oeuvre dans l'étape 6) du procédé de l'invention. A titre d'exemple, la recombinase peut être produite dans la cellule préalablement transfectée par l'intermédiaire d'un vecteur comportant un gène codant pour ladite recombinase.

Les cellules qui n'auront pas intégré le vecteur d'expression au niveau du site de reconnaissance d'une recombinase mourront, par conséquent, du fait des fortes doses d'antibiotique utilisées. En effet, le gène de résistance à l'antibiotique, qui ne dispose pas de séquence de polyadénylation en aval de sa séquence codante, ne pourra pas s'exprimer s'il n'est pas placé à proximité de la séquence de polyadénylation demeurée dans le génome des cellules à l'issu des étapes 3) et 4).

Avantageusement, la protéine d'intérêt issue de l'expression de l'ADN d'intérêt présent dans le vecteur d'expression peut être n'importe quelle protéine d'intérêt industriel ou thérapeutique. Elle peut être choisie par exemple parmi les anticorps, les facteurs de la coagulation, les cytokines, les facteurs de croissance, les enzymes, les hormones, cette liste n'étant pas limitative. Par ailleurs, le gène codant pour la protéine d'intérêt peut être le même que celui porté par le vecteur de ciblage ou être un gène codant pour une protéine différente.

Un autre objet de l'invention concerne une lignée cellulaire comportant dans son génome un site unique de reconnaissance d'une recombinase intégré dans une zone de forte activité transcriptionnelle, ladite lignée cellulaire ayant intégré une copie unique d'un transgène au niveau dudit site de reconnaissance d'une recombinase, ladite lignée cellulaire étant stable dans le temps, et susceptible d'être obtenue à l'issue de l'étape 4) du procédé de l'invention.

Avantageusement, cette lignée est la lignée cellulaire YGM-1/10G10, déposée le 18 Décembre 2006, sous le numéro de dépôt CNCM I-3704, auprès de la Collection Nationale de Culture de Microorganismes (CNCM). La lignée YGM-1/10G10 est obtenue par la mise en oeuvre du procédé de l'invention, en utilisant la cellule YB2/0 (ATCC CRL-1662) lors de l'étape 1) du procédé. Cette lignée cellulaire YGM-1/10G10 possède les caractéristiques suivantes : absence de séquences actives (promoteurs, gène de sélection, gène de résistance aux antibiotiques), présence d'un site de reconnaissance d'une recombinase, stabilité des paramètres de culture et du site d'intégration. Par ailleurs, cette lignée cellulaire présente avantageusement un taux de production de la protéine d'intérêt supérieur à 5 pcd (pg de protéine/cellule/24 heures). Avantageusement, le taux de production des cellules d'intérêt est supérieur à 10 pcd. De manière particulièrement avantageuse, le taux de production des cellules d'intérêt est supérieur à 15 pcd, et plus particulièrement à 20 pcd. Avantageusement, ce taux de production est compris entre 5 et 50 pcd, ou plus particulièrement entre 10 et 30 pcd.

Ainsi, un objet de l'invention est une nouvelle lignée d'expression YGM-1/10G10, libre de toute séquence transgénique active, dérivée de la lignée YB2/0, dans laquelle l'intégration du vecteur d'expression est contrôlée et dirigée dans une zone favorable à la transcription grâce à l'utilisation d'une recombinase.

Dans un autre mode de réalisation, cette lignée est la lignée cellulaire YGM-2/3G5, déposée le 19 Décembre 2007, sous le numéro de dépôt CNCM I-3885, auprès de la Collection Nationale de Culture de Microorganismes (CNCM). La lignée YGM-2/3G5 est obtenue par la mise en oeuvre du procédé de l'invention, en utilisant la cellule YB2/0 (ATCC CRL-1662) lors de l'étape 1) du procédé. Cette lignée cellulaire YGM-2/3G5 possède les caractéristiques suivantes : absence de séquences actives (promoteurs, gène de sélection, gène de résistance aux antibiotiques), présence d'un site de reconnaissance d'une recombinase, stabilité des paramètres de culture et du site d'intégration. Par ailleurs, cette lignée cellulaire présente avantageusement un taux de production de la protéine d'intérêt supérieur à 5 pcd (pg de protéine/cellule/24 heures). Avantageusement, le taux de production des cellules d'intérêt est supérieur à 10 pcd. De manière particulièrement avantageuse, le taux de production des cellules d'intérêt est supérieur à 15 pcd, et plus particulièrement à 20 pcd. Avantageusement, ce taux de production est compris entre 5 et 50 pcd, ou plus particulièrement entre 10 et 30 pcd.

Ainsi, un autre objet de l'invention est une nouvelle lignée d'expression YGM-2/3G5, libre de toute séquence transgénique active, dérivée de la lignée YB2/0, dans laquelle l'intégration du vecteur d'expression est contrôlée et dirigée dans une zone favorable à la transcription grâce à l'utilisation d'une recombinase.

Les lignées YGM-1/10G10 et YGM-2/3G5 présentent les caractéristiques et avantages suivants :
- Reproductibilité de l'expression, avec un niveau d'expression élevé et constant à chaque transfection grâce à l'intégration ciblée dans le même site ;
- Absence de séquences transgéniques actives (promoteurs, gènes de résistance, activateurs transcriptionnels) garantissant l'absence de modifications des caractéristiques de la lignée d'origine YB2/0 et plus de facilité d'utilisation lors de l'intégration ultérieure d'un vecteur d'expression d'une protéine d'intérêt ;
- Gain de temps et de ressources grâce au faible nombre de transfectants à cribler ;
- Possibilité de pratiquer une étape d'amplification génique (par exemple, par l'intermédiaire d'un système de type dhfr-méthotrexate) grâce à la sélection d'un site d'intégration « amplifiable ».

Avantageusement, la lignée cellulaire obtenue par le procédé de l'invention est stable au cours du temps sur une période d'au moins 3 mois, soit environ 80 temps de doublement.

Un autre objet de l'invention concerne une molécule d'acide nucléique isolée de séquence SEQ ID NO :1 comprenant un fragment d'acide nucléique de séquence SEQ ID NO :4, ledit fragment étant capable d'augmenter l'expression d'une protéine d'intérêt recombinante lorsque ledit fragment d'acides nucléiques est incorporé dans un vecteur d'expression, ou la zone de forte activité transcriptionnelle comportant la séquence SEQ ID NO : 1 ou un fragment d'acide nucléique comprenant au moins 80% d'homologie avec la séquence SEQ ID NO : 1, ledit fragment étant capable d'augmenter l'expression d'une protéine recombinante à laquelle on s'intéresse lorsque ledit fragment d'acides nucléiques est incorporé dans un vecteur d'expression.

La séquence d'acide nucléique correspondant à la molécule d'acide nucléique isolée de séquence SEQ ID NO : 1 comprenant un fragment d'acide nucléique capable d'augmenter l'expression d'une protéine d'intérêt recombinante lorsque ledit fragment d'acides nucléiques est incorporé dans un vecteur d'expression est la suivante :

La molécule d'acide nucléique isolée de séquence SEQ ID NO : 1 comprenant un fragment d'acide nucléique capable d'augmenter l'expression d'une protéine d'intérêt recombinante lorsque ledit fragment d'acides nucléiques est incorporé dans un vecteur d'expression dont la séquence est présentée ci-dessus possède les caractéristiques suivantes (les positions sont données en fonction de la numérotation des nucléotides composant la séquence, laquelle numérotation est détaillée ci-dessus) :
- de la position 1 à la position 1024 : séquence génomique ;
- de la position 2631 à la position 2678 : site Frt ;
- de la position 1025 à la position 4419 : séquence du vecteur de ciblage restant en place après délétion.

La séquence d'acide nucléique correspondant au fragment d'acide nucléique capable d'augmenter l'expression d'une protéine d'intérêt recombinante lorsque ledit fragment d'acides nucléiques est incorporé dans un vecteur d'expression est la suivante (SEQ ID NO : 4) :

La présente demande décrit également un vecteur comportant la séquence d'acides nucléiques SEQ ID NO : 1.

Un autre objet de l'invention est une lignée cellulaire comportant dans son génome un site unique de reconnaissance de recombinase intégré dans une zone de forte activité transcriptionnelle, ladite lignée cellulaire ayant intégré une seule copie d'un transgène au niveau dudit site de reconnaissance de recombinase, ladite lignée cellulaire étant stable dans le temps, et susceptible d'être obtenue par le procédé de l'invention.

Une telle lignée comporte deux séquences en tandem correspondant à des sites de reconnaissance d'une recombinase de part et d'autre d'un gène codant pour une protéine d'intérêt et d'un gène de résistance à un antibiotique, se situant immédiatement en amont du site de reconnaissance d'une recombinase le plus en aval, la séquence de polyadénylation du gène de résistance à l'antibiotique se situant immédiatement en aval de ce site de reconnaissance à l'antibiotique.

Avantageusement, cette lignée cellulaire possède, avant amplification génique, une productivité dudit transgène comprise entre 5 et 50 pcd (pg de protéines/cellule/24h).

Avantageusement, cette lignée cellulaire exprime ledit transgène de manière stable sur une période d'au moins 3 mois.

Un autre objet de l'invention concerne un procédé de production de protéines d'intérêt, dans lequel on met en culture la lignée cellulaire de l'invention exprimant une protéine d'intérêt, de manière à exprimer ladite protéine d'intérêt, et à récupérer ladite protéine d'intérêt.

La présente demande décrit un vecteur pour l'insertion d'un ADN d'intérêt dans le génome d'une cellule de mammifère (« vecteur de ciblage ») , comportant deux sites de reconnaissance d'une recombinase situés de part et d'autre d'un gène rapporteur, d'un gène codant pour une protéine d'intérêt, d'un gène de sélection permettant l'amplification génique et d'un gène de résistance à un antibiotique, et une séquence de polyadénylation située en aval du deuxième site de reconnaissance d'une recombinase. Avantageusement, la séquence de polyadénylation du gène de résistance à l'antibiotique est un polyA faible. Ceci permet d'obtenir de meilleurs taux de production en sortie de criblage.

Avantageusement, ce vecteur comporte, entre les deux sites de reconnaissance de recombinase, un gène codant pour la thymidine kinase du virus Herpès simplex de type I (HSV1-TK).

La présente demande de brevet décrit également un système de vecteurs pour l'insertion d'un ADN d'intérêt à un site cible dans le génome d'une cellule de mammifère, qui comprend au moins les constituants suivants :
- un vecteur de ciblage tel que décrit précédemment,
- un vecteur d'expression comprenant un site de reconnaissance d'une recombinase, un gène codant pour une protéine d'intérêt et un gène de résistance à un antibiotique, le site de reconnaissance à la recombinase étant situé directement en aval du gène de résistance à l'antibiotique.

La présente demande de brevet décrit également l'utilisation du système de vecteur précité dans un procédé pour insérer une molécule d'ADN d'intérêt au sein d'un site cible dans le génome d'une cellule de mammifère, comprenant les étapes suivantes :
- la transfection d'une cellule de mammifère avec ledit vecteur de ciblage ;
- la sélection des cellules « fortement productrices » ayant intégré une seule copie du vecteur de ciblage ;
- l'excision du vecteur de ciblage par l'action de la recombinase ;
- la sélection des cellules ayant excisé le vecteur de ciblage et possédant un site de reconnaissance intègre ;
- la transfection des cellules sélectionnées à l'étape 4) avec ledit vecteur d'expression ;
- l'intégration du vecteur d'expression dans le site de reconnaissance intègre par l'action de la recombinase ;
   la sélection des cellules contenant le vecteur d'expression intégré au site cible en testant l'expression de la protéine d'intérêt. Avantageusement, la sélection sera réalisée en présence d'une forte dose d'antibiotique afin de défavoriser les intégrations aléatoires.

D'autres aspects et avantages de l'invention seront décrits dans les exemples qui suivent, qui doivent être considérés comme illustratifs et ne limitent pas l'étendue de l'invention.

### EXEMPLES

### Exemple 1 : Réalisation des vecteurs utilisés pour la fabrication de la lignée cellulaire dans laquelle un site unique de reconnaissance d'une recombinase est intégré dans une zone de forte activité transcriptionnelle

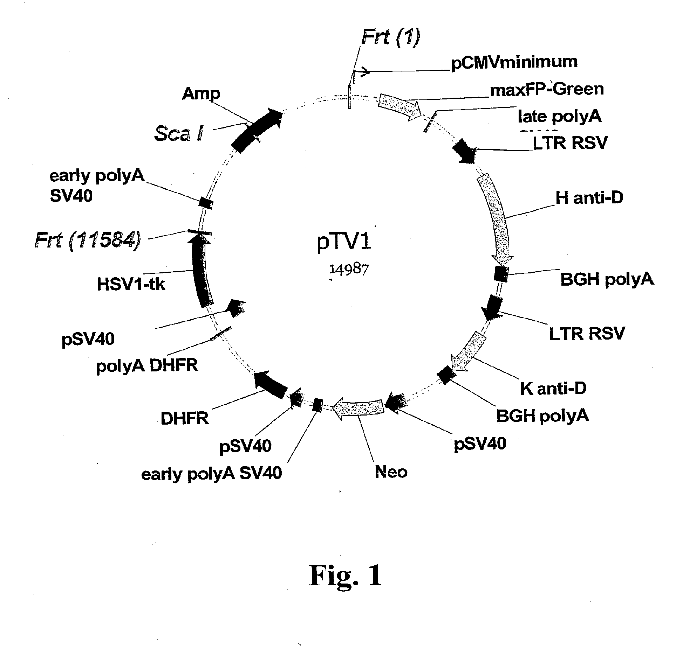

### a. Vecteur de ciblage pTV1 (voir figure 1):

- Unités de transcription : Le vecteur de ciblage pTV1 a été construit (SEQ ID NO : 2) (Fig.1) qui contient les unités de transcription suivantes :
   1- l'unité de transcription de maxFP-Green (gène rapporteur fluorescent) contenant dans l'ordre le promoteur minimum CMV (promoteur du gène précoce du cytomégalovirus humain sans sa partie activatrice ou enhancer), le gène codant la protéine maxFP™-Green (Evrogen) et la séquence de polyadénylation (polyA) late polyA SV40 (virus simien 40),
   2- l'unité de transcription d'une chaîne lourde d'anticorps anti-D contenant dans l'ordre un promoteur RSV (Long Terminal Repeat du Virus du Sarcome de Rous), un intron artificiel issu du vecteur pCi-néo, la séquence de la chaîne lourde d'immunoglobuline anti-D et la séquence du polyA BGH (Bovine growth Hormone),
   3- l'unité de transcription d'une chaîne légère d'anticorps anti-D contenant les mêmes éléments que l'unité de transcription de la chaîne lourde de l'anticorps à l'exception de la séquence de la chaîne légère d'immunoglobuline et la séquence du polyA BGH.
   4- l'unité de transcription néo contenant le promoteur SV40, le gène de résistance à la néomycine et la séquence « early polyA SV40 » possédant une activité polyA « faible »,
   5- l'unité de transcription DHFR (dihydrofolate réductase) contenant le promoteur SV40, le gène de sélection dhfr modifié par mutagénèse dirigée afin d'éliminer le site de restriction ScaI (mutation silencieuse au niveau protéique) et son site de polyA,
   6- l'unité de transcription HSV1-tk contenant le promoteur SV40, le gène suicide codant la thymidine kinase du virus Virus Herpes simplex de type 1 (HSV1-tk) et la séquence « early polyA SV40 ».
- Synthèse des sites de recombinaison *Frt* :

Les sites de recombinaison *Frt* sont synthétisés par PCR grâce aux amorces suivantes :
Amorces sens (SEQ ID No : 5) :
   5'-ACAGCTGTCGACTGAAGTACCTATTCCGAAGTTCCTATTCTCTAGAAAGT-3'
Amorces antisens (SEQ ID No :6) :
   5'-CGTCCGGATATCTAAGATCTGAAGTTCCTATACTTTCTAGAGAATAGGAA-3'

Le produit de PCR obtenu contient le site Frt (en gras et italique) ainsi que des sites de restrictions (soulignés) permettant le clonage par la suite dans le vecteur de ciblage pTV1 (SEQ ID No : 7):

Un premier site Frt *(Frt1)* a été cloné en amont de l'unité de transcription maxFP-Green du vecteur pTV1. Un second Site Frt *(Frt2)* a été cloné dans le même sens que *Frt*1 entre le gène HSV1-tk et son site de polyadénylation « early polyA SV40 ».

La séquence d'acide nucléique correspondant au vecteur de ciblage pTV1 est la suivante (SEQ ID NO : 2) :

Le vecteur de ciblage pTV1 dont la séquence est présentée ci-dessus (SEQ ID NO: 2) possède les caractéristiques suivantes (les positions sont données en fonction de la numérotation des nucléotides composant la séquence, laquelle numérotation est détaillée ci-dessus) :
- de la position 1 à la position 48 : site de recombinase FRT ;
- de la position 63 à la position 144 : promoteur CMV minimum ;
- de la position 478 à la position 1176 : protéine maxFP-green ;
- de la position 1352 à la position 1402 : signal de polyadénylation SV40 late ;
- de la position 1888 à la position 2283 : LTR RSV ;
- de la position 2521 à la position 3951 : chaîne lourde (H) Ig anti-D ;
- de la position 3958 à la position 4184 : signal de polyadénylation bGH ;
- de la position 4431 à la position 4826 : LTR RSV ;
- de la position 5064 à la position 5771 : chaîne légère Kapppa (K) Ig anti-D ;
- de la position 5778 à la position 6004 : signal de polyadénylation bGH ;
- de la position 6658 à la position 6983 : promoteur SV40 ;
- de la position 7019 à la position 7813 : séquence codante neo phosphotransférase ;
- de la position 7987 à la position 8117 : signal de polyadénylation précoce de SV40 (early polyA SV40);
- de la position 8301 à la position 8496 : promoteur SV40 ;
- de la position 8595 à la position 9158 : séquence codante DHFR ;
- de la position 9965 à la position 10019 : signal de polyadénylation SV40 late ;
- de la position 10092 à la position 10394 : promoteur SV40 ;
- de la position 10442 à la position 11572 : séquence codante HSV-TK ;
- de la position 11584 à la position 11631 : site de recombinase FRT ;
- de la position 11974 à la position 12104 : signal de polyadénylation précoce de SV40 (early polyA RSV40);
- de la position 12996 à la position 13926 : gène de résistance à l'ampicilline.

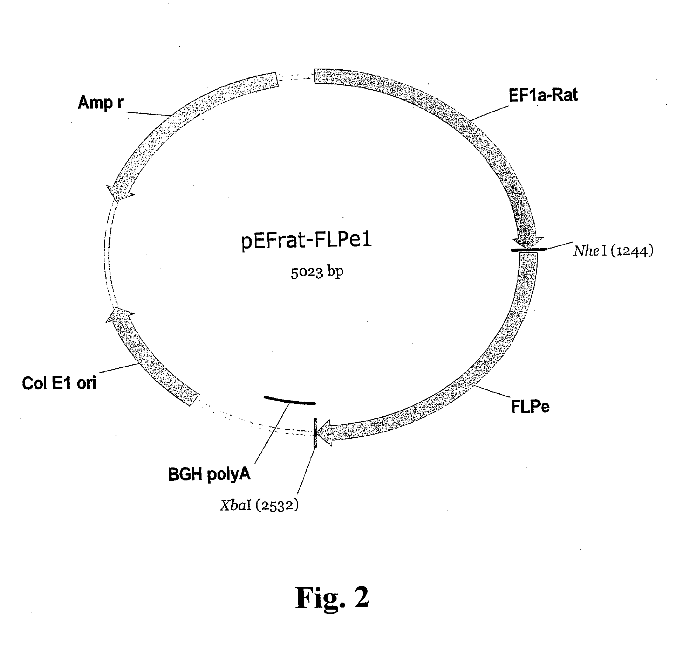

### b. Vecteur pEFrat-FLPe (SEQ ID NO . 3, Fig. 2) :

Le gène codant la recombinase FLPe a été amplifié par PCR à partir du vecteur pOG4-FLPe (55) avec les amorces suivantes :
Amorce sens (SEQ ID No : 8) :
   5'-ATCTGGCTAGCCGCCACC***ATG*CCACAATTTGATATATTAT**-3'

La séquence soulignée correspond au site de restriction NheI. En gras est représenté le début de la partie codante du gène FLPe avec l'ATG initiateur en italique.

Amorces antisens (SEQ ID No : 9) :
5'-TGTCATCTAGATTA***TTA*TATGCGTCTATTTATGT**-3'

La séquence soulignée correspond au site de restriction XbaI. En gras est représenté la fin de la séquence codante du gène FLPe avec le codon STOP en italique.

Le produit de PCR FLPe ainsi obtenu a été cloné entre le promoteur EF1-alpha de rat et une séquence de polyadénylation bGH (bovine growth hormone) au niveau des sites NheI et XbaI pour obtenir le vecteur d'expression final pEFrat-FLPe.

La séquence d'acide nucléique correspondant au vecteur pEFrat-FLPe est la suivante (SEQ ID NO : 3) :

Le vecteur pEFrat-FLPe dont la séquence est présentée ci-dessus (SEQ ID NO: 3) possède les caractéristiques suivantes (les positions sont données en fonction de la numérotation des nucléotides composant la séquence, laquelle numérotation est détaillée ci-dessus) :
- de la position 1 à la position 1243 : promoteur EF-1alpha rat ;
- de la position 1256 à la position 2527 : séquence codante Flpe ;
- de la position 2537 à la position 2763 : signal de polyadénylation bGH ;
- de la position 3020 à la position 3543 : origine de réplication Col E1 ;
- de la position 4887 à la position 4027 : gène de résistance à l'ampicilline.

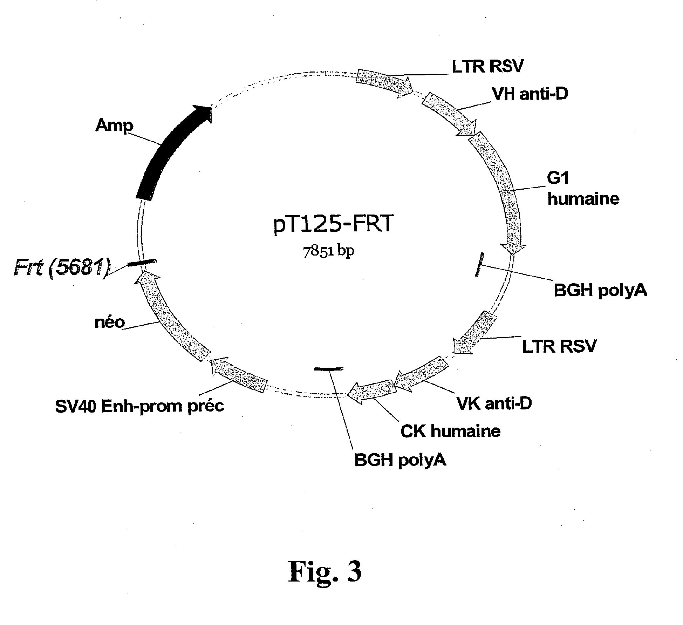

### c. Vecteur de réintégration pT125-FRT (Fig. 3):

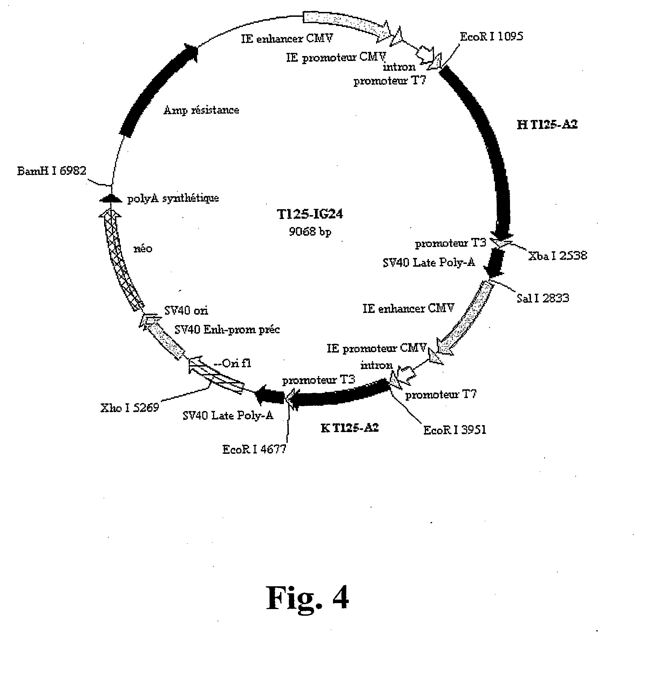

Le vecteur de réintégration pT125-FRT est issu du vecteur T125-IG24 (Fig. 4) dans lequel les promoteurs CMV ont été remplacés par des promoteurs RSV et dans lequel la séquence de polyadénylation du gène de résistance à la néomycine à été enlevée et remplacée par un site Frt placé dans la même orientation que les sites Frt du vecteur pTV1.

### Exemple 2 : Ciblage de zones à haute activité transcriptionnelle dans la lignée YB2/0

### a. Transfection de la lignée YB2/0 par le vecteur pTV1

La lignée de rat YB2/0 (ATCC # CRL-1662) a été cultivée en milieu EMS (Invitrogen, ref. 041-95181M) contenant 5% de sérum de veau foetal (SVF). 5 millions de cellules ont été électroporées (électroporateur BioRad, modèle 1652077) en utilisant le kit de transfection Optibuffer (Thermo Electron). Pour chaque transfection, 5µg de vecteur pTV1 linéarisé par l'enzyme de restriction ScaI ont été utilisés. Les conditions d'électroporation appliquées étaient de 230 volts et 950 microfarads dans une cuvette de 0,5ml. Le contenu de chaque cuvette d'electroporation a ensuite été réparti dans 10 plaques P96 avec une densité de 5000 cellules par puit. La mise en milieu sélectif a été réalisée 3 jours après la transfection dans du milieu EMS contenant 5% de SVF et 2mg/ml de G418 (Invitrogen, ref. 10131-027).

### b. Criblage par fluorescence des clones résistants au G418

10 jours et 15 jours après la transfection, les clones résistants ont été criblés par fluorescence induite par la protéine maxFP™14-Green (longueur d'onde d'excitation 482nm, émission 502nm). Une première lecture a été effectuée sur tous les clones résistants dans un lecteur de plaques à fluorescence (VICTOR3, Perkin Elmer). Tous les clones positifs en fluorescence ont alors été testés par cytométrie en flux afin de vérifier l'intensité de fluorescence propre à chaque cellule et l'homogénéité des clones. Les résultats ont été analysés sur le logiciel libre WinMDI 2.8 (http://facs.scripps.edu). Tous les clones présentant un pic de fluorescence homogène et une intensité moyenne de fluorescence (MFI) supérieure à 500 ont été conservés et passés en plaques P24 pour amplification.

### c. Criblage par ELISA et détermination du nombre de copies

Les cellules ont été entretenues en plaques P24 et leur surnageant a été dosé en ELISA afin d'estimer leur productivité en anticorps Anti-D. Tous les clones présentant une productivité supérieure à 10 pcd (picogrammes/cellule/24h) ont alors été testés en PCR semi-quantitative afin d'estimer le nombre de copies du vecteur pTV1. L'estimation du nombre de copie du vecteur pTV1 a été réalisée dans un appareil ABI PRISM® 7000 avec comme témoin normalisateur une PCR sur les séquences LINE (séquences répétées présentant en nombre stable dans les clones obtenus à partir de la lignée YB2/0). Les clones estimés à une seule copie ont alors été testés en Southern blot pour confirmation du nombre de copie.

### d. Test d'amplification génique au méthotrexate (MTX)

Des tests d'amplification génique au MTX (sigma, ref. M8407) ont été réalisés sur les clones forts producteurs à copie unique afin de déterminer les capacités d'amplification du locus ciblé par le vecteur pTV1. Pour cela, des doses de MTX supérieures ou égales à 25 nM ont été ajoutées au milieu EMS, 5% SVF. Après 15 jours de culture en milieu sélectif, les clones résistants ont été testés en ELISA afin de vérifier l'augmentation de productivité et en PCR quantitative afin de déterminer l'augmentation du nombre de copie. La productivité du clone 3G11 (9,7 pcd) a été ainsi augmentée à 22 pcd après un seul cycle amplification. De même la productivité du cloïde 35H4 (11,4 pcd) est augmentée d'environ 3 fois après un cycle d'amplification.

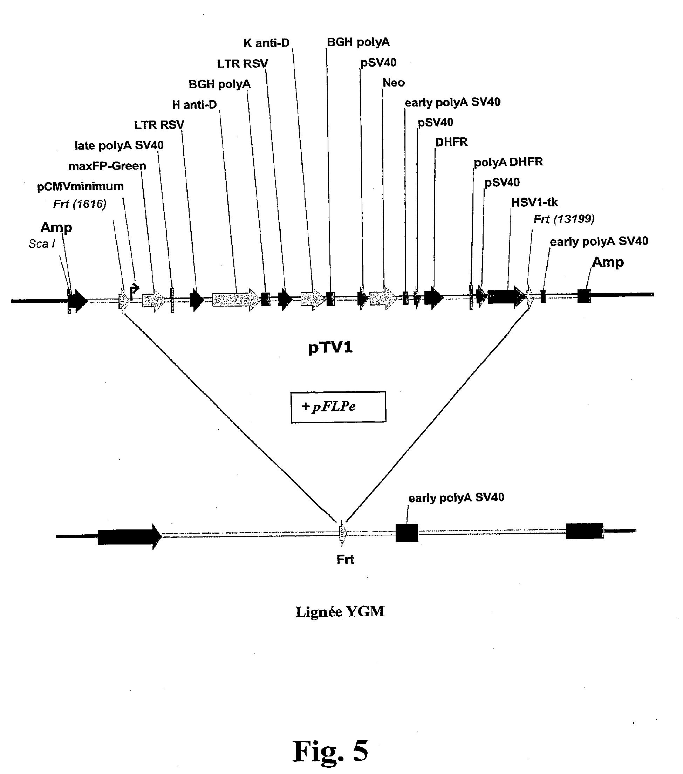

### Exemple 3 : Excision du vecteur de ciblage pTV1 par action de la recombinase FLPe, obtention des lignées YGM-1/10G10 et YGM-2/3G5 (Fig. 5)

### • Lignée YGM-1/10G10

Les clones 8A10 et 3G11, forts producteurs (6,6 pcd et 9,7 pcd respectivement sans amplification) à copie unique de vecteur pTV1 ont été sélectionnés pour générer une lignée YGM par délétion du vecteur de ciblage.

Le clone 8A10 a été tout d'abord amplifié dans des flasques T75 en milieu EMS, 5% SVF, 2mg/ml G418 . La veille de la transfection, le G418 a été retiré de la culture.

5µg du vecteur pEFrat-FLPe non linéarisé dilué dans 175µl d'EMS sans sérum et 25µl de Superfect ont été incubés à température ambiante 10 minutes puis mélangés avec 1ml d'EMS, 5% SVF. Ce mélange a été rajouté à 2x10⁶ cellules préalablement lavées en PBS et diluées à 2,5x10⁵ cellules/ml dans du milieu EMS, 5% SVF. Après 4 heures d'incubation à 37°C, les cellules ont été passées en milieu EMS, 5% SVF.

Après 24 heures de culture, les cellules ont été réparties en plaques P96 à une densité de 5000 cellules par puit.

La mise en milieu sélectif a été réalisée 2 jours après la transfection dans du milieu EMS, 5% SVF contenant 4µM de Ganciclovir (Invivogen, ref. sud-gcv).

Après 15 jours de culture, les clones survivants ont été testés pour l'absence de fluorescence en cytométrie de flux, pour l'absence de production d'immunoglobulines anti-D en ELISA et pour l'excision complète du vecteur pTV1 en PCR avec les amorces suivantes :
5'pTV1 (SEQ ID No : 10) : 5'-CCTATGGAAAAACGCCAGCAAC-3'
3'pTV1 (SEQ ID No : 11) : 5'-CCTTAGAAAGCGGTCTGTGAAA-3'

Parmi les clones excisés, le clone 10G10 a été sélectionné pour constituer la lignée d'expression YGM-1/10G10 et contrôlé pour la stabilité du site d'intégration sur une période de 3 mois.

### • Lignée YGM-2/3G5

Dérivé du cloïde 35H4, fort producteur (11,4 pcd sans amplification) à copie unique de vecteur pTV1, le clone 35H4(1)2G2 (13,3 pcd) a été sélectionné pour générer une lignée YGM par délétion du vecteur de ciblage.

Le clone 35H4(1)2G2 a été tout d'abord amplifié dans des flasques T75 en milieu RPMI, 5% SVF, 1mg/ml G418. Le G418 a été retiré de la culture deux repiquages avant la transfection.

25µg du vecteur pEFrat-FLPe non linéarisé dilué dans 500µl de RPMI sans sérum et 75µl de FuGene HD (Roche) ont été incubés à température ambiante 15 minutes. 120µl de ce mélange a été rajouté dans chaque puits de plaque P6 contenant 6x10⁵ cellules/ml dans du milieu RPMI, 5% SVF. Après 24 heures de culture, les cellules ont été réparties en plaques P96 à une densité de 1000 cellules par puits.

La mise en milieu sélectif a été réalisée 3 jours après la transfection dans du milieu RPMI, 5% SVF contenant 4µM de Ganciclovir (Invivogen, ref. sud-gcv).

Après 15 jours de culture, les clones survivants ont été testés pour l'absence de fluorescence en cytométrie de flux, pour l'absence de production d'immunoglobulines anti-D en ELISA et pour l'excision complète du vecteur pTV1 en PCR avec les amorces suivantes :
DEL REV (SEQ ID No : 14): 5'-TGGTATGGCTGATTATGATCCTC-3'
DEL FOR 3 (SEQ ID No : 15): 5'-CCTTTTGCTCACATGGCTCGAC-3'

Parmi les clones excisés, le clone 35H4(2)3G5 a été sélectionné pour constituer la lignée d'expression YGM-2/3G5 et contrôlé pour la stabilité du site d'intégration sur une période de 3 mois.

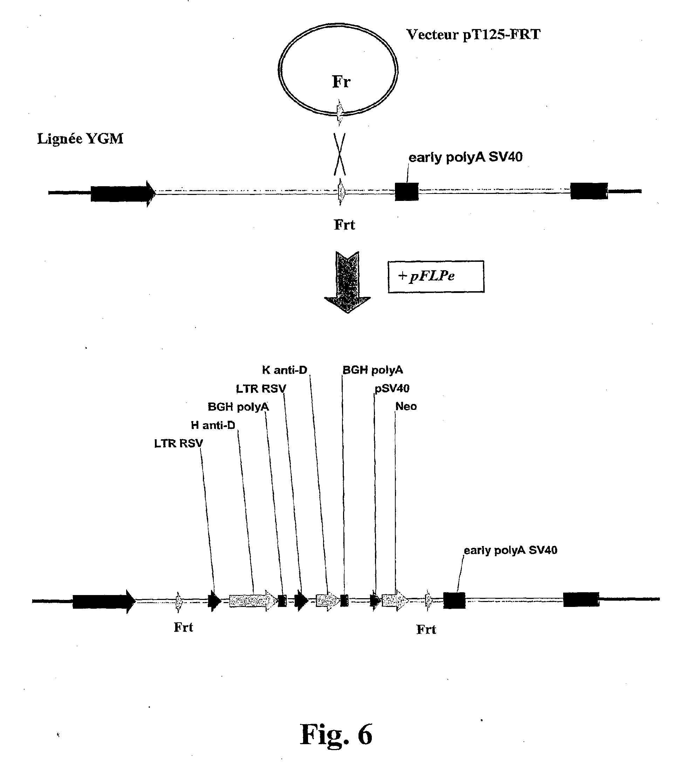

### Exemple 4 : Réintégration d'un vecteur d'expression dans les lignées YGM-1/10G10 et YGM-2/3G5(Fig. 6)

### • Lignée YGM-1/10G10

La réintégration d'un vecteur codant le même anticorps utilisé lors de l'étape de criblage (anticorps anti-D) a été réalisée afin de vérifier la reproductibilité des niveaux d'expression après réintégration dans le site Frt de YGM-1 (clone 10G10).

Le clone 10G10 obtenu après excision du vecteur de ciblage TV1 et ne possédant dans son génome qu'un site de recombinaison Frt et une séquence de polyadénylation « early polyA SV40 » a été amplifié dans des flasques T75 en milieu EMS, 5% SVF.

Une cotransfection a été réalisée avec 10 µg du vecteur pEFrat-FLPe non linéarisé et 5 µg de vecteur de réintégration pT125-FRT non linéarisé. 5 millions de cellules ont été électroporées dans une cuvette de 0,5ml (électroporateur BioRad, modèle 1652077) en utilisant le kit de transfection Optibuffer (Thermo Eélectron) et en applicant les conditions suivantes : 230 volts et 950 microfarads.

Les cellules ont été ensuite réparties sur 10 plaques P96 à une densité de 5000 cellules par puit.

La mise en milieu sélectif a été réalisée 2 jours après la transfection dans du milieu EMS, 5% SVF contenant 2mg/ml de G418.

Après 15 jours de culture, les clones survivants ont été amplifiés en plaques P24 et criblés en PCR afin de vérifier la réintégration dans le site Frt de la lignée YGM-1.

Les amorces m5NEO-2 et SV40polyA-1rev ont été utilisées pour ce criblage :
Amorce m5NEO-2 (SEQ ID No 12) :
   5'-GATGCCTGCTTGCCGAATA-3'
Amorce SV40polyA-1rev (SEQD ID No : 13):
   5'-CCTTAGAAAGCGGTCTGTGAAA-3'

Les clones présentant des intégrations aléatoires dans le génome de la lignée YGM-1 ont été éliminés. Les clones ayant intégré le vecteur pT125-FRT dans le site Frt de la lignée YGM-1 ont alors été testés en ELISA afin d'évaluer leur productivité en anticorps Anti-D T125.

Le clone 21B10 (5.5 pcd) a été retenu pour vérifier la stabilité de la réintégration sur une période de 3 mois.

### • Lignée YGM-2/3G5

La réintégration d'un vecteur codant le même anticorps utilisé lors de l'étape de criblage (anticorps anti-D) a été réalisée afin de vérifier la reproductibilité des niveaux d'expression après réintégration dans le site Frt de YGM-2 (clone 35H4 (2) 3G5).

Le clone 35H4(2)3G5 obtenu après excision du vecteur de ciblage TV1 et ne possédant dans son génome qu'un site de recombinaison Frt et une séquence de polyadénylation « early polyA SV40 » a été amplifié dans des flasques T75 en milieu RPMI, 5% SVF.

Une cotransfection a été réalisée avec 4 µg du vecteur pEFrat-FLPe non linéarisé et 2 µg de vecteur de réintégration pT125-FRT non linéarisé.

Les deux vecteurs non linéarisés dilués dans 300µl de EMS sans sérum et 18µl de FuGene HD (Roche) ont été incubés à température ambiante 15 minutes. 75µl de ce mélange a été rajouté dans chaque puits de plaque P6 contenant 6x10⁵ cellules/ml dans du milieu EMS, 5% SVF. Après 24 heures de culture, les cellules ont été réparties en plaques P96 à une densité de 1000 cellules par puits.

Les cellules ont été ensuite réparties sur 10 plaques P96 à une densité de 1000 cellules par puits.

La mise en milieu sélectif a été réalisée 2 jours après la transfection dans du milieu EMS, 5% SVF contenant 3mg/ml de G418.

Après 12 jours de culture, les clones survivants ont été amplifiés en plaques P24 et criblés en PCR afin de vérifier la réintégration dans le site Frt de la lignée YGM-2.

Les amorces suivantes ont été utilisées pour ce criblage.
- Contrôle de l' intégration en 5'
   DEL FOR 3 (SEQ ID No : 15) : 5'- CCTTTTGCTCACATGGCTCGAC -3'
   3FRT3 (SEQ ID No : 16) : 5'- TTGTCTCATGAGCGGATACA -3'
- Contrôle de l' intégration en 3'
   DEL REV (SEQ ID No : 14) : 5'- TGGTATGGCTGATTATGATCCTC -3'
   m-5-NEO-2 (SEQ ID No : 12) : 5'- GATGCCTGCTTGCCGAATA -3'

Grâce à ce criblage les transfectants résistants au G418 mais présentant des intégrations aléatoires dans le génome de la lignée YGM-2 ont été éliminés. Parmi les transfectants ayant intégré le vecteur pT125-FRT dans le site Frt de la lignée YGM-2 les cloïdes 19D1, 25E5, 30A5 et 20F11 ont été testés en ELISA afin d'évaluer leur productivité en anticorps Anti-D. Les productivités obtenues pour ces 4 clones (7,1 pcd; 7,0 pcd ; 7,1 pcd et 7,7 pcd, respectivement) sont homogènes et du même ordre de grandeur que celle observées pour le cloïde 35H4 ou le clone 35H4(1)2G2 parental (11,4 pcd et 13,3 pcd, respectivement) témoignant de l'intérêt de cette stratégie de ciblage pour l'obtention de forts producteurs de façon reproductible. La stabilité génétique de ces cloïdes a été étudiée, après clonage, sur une période de 3 mois.

### Exemple 5 : identification du site d'intégration de la lignée YGM-1 (clone 10G10) par PCR-inverse

La caractérisation de la séquence du site d'intégration a été réalisée après amplification par PCR-inverse. Brièvement, l'ADN des clones 8A10 (contenant le vecteur de ciblage) ou 10G10 (lignée YGM-1 délétée) a été digéré par différentes enzymes de restriction et les fragments de restriction obtenus ont été religaturés sur eux-même à l'aide d'une ligase T4. Des PCR-inverse ont été ensuite réalisées en utilisant des amorces dirigées en sens opposé et ancrées dans les séquences du vecteur de ciblage. Les produits de PCR ainsi obtenus présentent à leurs extrémités 5' et 3' des séquences issues du vecteur de ciblage et en leur centre une séquence inconnue de taille variable (en fonction de l'enzyme de restriction utilisée) correspondant à la région d'intégration directement adjacente au vecteur de ciblage. Le séquençage de ces différents produits de PCR permet ainsi d'obtenir la séquence du site d'intégration à forte activité transcriptionnelle (SEQ ID NO :1).

### Références

1. Baneyx F. and M. Mujacic. Recombinant protein folding and misfolding in Escherichia coli. Nat Biotech 22 (11):1399-1408, 2004.
2. Makrides, S. C. 1996. Strategies for achieving high-level expression of genes in Escherichia coli. Microbiol. Rev 60:512.
3. Gasser, B., and D. Mattanovich. 2006. Antibody production with yeasts and filamentous fungi: on the road to large scale? Biotechnol Lett.
4. T. U. Gerngross. Advances in the production of human therapeutic proteins in yeasts and filamentous fungi. Nat Biotech 22 (11):1409-1414, 2004.
5. Smerdon, G. R., E. F. Walton, and S. J. Aves. 1998. Stable production of human gastric lipase by chromosomal integration in the fission yeast Schizosaccharomyces pombe. Appl. Microbiol. Biotechnol 49:45.
6. Hellwig, S., J. Drossard, R. M. Twyman, and R. Fischer. 2004. Plant cell cultures for the production of recombinant proteins. Nat Biotechnol 22:1415.
7. Miller, L. K. 1993. Baculoviruses: high-level expression in insect cells. Curr Opin Genet. Dev 3:97.
8. McCall, E. J., A. Danielsson, I. M. Hardern, C. Dartsch, R. Hicks, J. M. Wahlberg, and W. M. Abbott. 2005. Improvements to the throughput of recombinant protein expression in the baculovirus/insect cell system. Protein Expr. Purif. 42:29.
9. Schmidt, F. R. 2004. Recombinant expression systems in the pharmaceutical industry. Appl Microbiol. Biotechnol 65:363.
10. Simonsen, C. C., and M. McGrogan. 1994. The molecular biology of production cell lines. Biologicals 22:85.
11. Geisse, S., H. Gram, B. Kleuser, and H. P. Kocher. 1996. Eukaryotic expression systems: a comparison. Protein Expr. Purif. 8:271.
12. Bendig, M. M. 1988. The production of foreign proteins in mammalian cells. Genet. Eng91.C.
13. Gorman and C. Bullock. Site-specific gene targeting for gene expression in eukaryotes. Curr Opin Biotechnol 11 (5):455-460, 2000.
14. M. E. Reff. High-level production of recombinant immunoglobulins in mammalian cells. Curr Opin Biotechnol 4 (5):573-576, 1993.
15. D. C. Andersen and L. Krummen. Recombinant protein expression for therapeutic applications. Curr Opin Biotechnol 13 (2):117-123, 2002.
16. R. J. Kaufman. Use of recombinant DNA technology for engineering mammalian cells to produce proteins. Bioprocess Technol 10:15-69, 1990.
17. R. J. Kaufman. Overview of vector design for mammalian gene expression. Mol.Biotechnol 16 (2):151-160, 2000.
18. Lucas, B. K., L. M. Giere, R. A. DeMarco, A. Shen, V. Chisholm, and C. W. Crowley. 1996. High-level production of recombinant proteins in CHO cells using a dicistronic DHFR intron expression vector. Nucl. Acids Res. 24:1774.
19. F. Hesse and R. Wagner. Developments and improvements in the manufacturing of human therapeutics with mammalian cell cultures. Trends Biotechnol 18 (4):173-180, 2000.
20. M. Butler. Animal cell cultures: recent achievements and perspectives in the production of biopharmaceuticals. Appl Microbiol.Biotechnol 68 (3) :283-291, 2005.
21. Fussenegger, M., and J. E. Bailey. 1998. Molecular regulation of cell-cycle progression and apoptosis in mammalian cells: implications for biotechnology. Biotechnol Prog. 14:807.
22. Peakman, T. C., J. Worden, R. H. Harris, H. Cooper, J. Tite, M. J. Page, D. R. Gewert, M. Bartholemew, J. S. Crowe, and S. Brett. 1994. Comparison of expression of a humanized monoclonal antibody in mouse NSO myeloma cells and Chinese hamster ovary cells. Hum Antibodies Hybridomas 5:65.
23. S. J. Kim, N. S. Kim, C. J. Ryu, H. J. Hong, and G. M. Lee. Characterization of chimeric antibody producing CHO cells in the course of dihydrofolate reductase-mediated gene amplification and their stability in the absence of selective pressure. Biotechnol Bioeng. 58 (1):73-84, 1998.
24. Seth, G., P. Hossler, J. C. Yee, and W. S. Hu. 2006. Engineering cells for cell culture bioprocessingphysiological fundamentals. Adv. Biochem. Eng Biotechnol 101:119.
25. F. M. Wurm, A. Johnson, T. Ryll, C. Kohne, H. Scherthan, F. Glaab, Y. S. Lie, C. J. Petropoulos, and W. R. Arathoon. Gene transfer and amplification in CHO cells. Efficient methods for maximizing specific productivity and assessment of genetic consequences. Ann.N.Y.Acad.Sci 782:70-78, 1996.
26. F. M. Wurm. Production of recombinant protein therapeutics in cultivated mammalian cells. Nat Biotechnol 22 (11):1393-1398, 2004.
27. Trill, J.J., A. R. Shatzman, and S. Ganguly. 1995. Production of monoclonal antibodies in COS and CHO cells. Curr Opin Biotechnol 6:553.
28. F. M. Wurm. Integration, amplification and stability of plasmid sequences in CHO cell cultures. Biologicals 18 (3):159-164, 1990.
29. M. Schroder, K. Matischak, and P. Friedl. Serum- and protein-free media formulations for the Chinese hamster ovary cell line DUKXB11. J Biotechnol 108 (3):279-292, 2004.
30. C. R. Bebbington, G. Renner, S. Thomson, D. King, D. Abrams, and G. T. Yarranton. High-level expression of a recombinant antibody from myeloma cells using a glutamine synthetase gene as an amplifiable selectable marker. Biotechnology (N.Y.) 10 (2):169-175, 1992.
31. Barnes LM et al 2000 Cytotechnology 32 : 109-123. Advances in animal cell recombinant protein production
32. Thomas, P., and T. G. Smart. 2005. HEK293 cell line: a vehicle for the expression of recombinant proteins. J Pharmacol. Toxicol. Methods 51:187.
33. Grunberg, J., K. Knogler, R. Waibel, and I. Novak-Hofer. 2003. High-yield production of recombinant antibody fragments in HEK-293 cells using sodium butyrate. Biotechniques 34:968.
34. Racher, A. J., J. L. Moreira, P. M. Alves, M. Wirth, U. H. Weidle, H. Hauser, M. J. Carrondo, and J. B. Griffiths. 1994. Expression of recombinant antibody and secreted alkaline phosphatase in mammalian cells. Influence of cell line and culture system upon production kinetics. Appl. Microbiol. Biotechnol 40:851.
35. Griffiths, J. B., and A. J. Racher. 1994. Cultural and physiological factors affecting expression of recombinant proteins. Cytotechnology 15:3.
36. Y. Kariya, K. Ishida, Y. Tsubota, Y. Nakashima, T. Hirosaki, T. Ogawa, and K. Miyazaki. Efficient expression system of human recombinant laminin-5. J Biochem.(Tokyo) 132 (4):607-612, 2002.
37. al-Shawi, R., J. Kinnaird, J. Burke, and J. O. Bishop. 1990. Expression of a foreign gene in a line of transgenic mice is modulated by a chromosomal position effect. Mol Cell Biol 10:1192.
38. Yoshimura, F. K., and K. Chaffin. 1987. Different activities of viral enhancer elements before and after stable integration of transfected DNAs. Mol Cell Biol 7:1296.
39. Felsenfeld, G., J. Boyes, J. Chung, D. Clark, and V. Studitsky. 1996. Chromatin structure and gene expression. PNAS 93:9384.
40. Guglielmi, L., V. Truffinet, M. Cogne, and Y. Denizot. 2003. The beta-globin HS4 insulator confers copy-number dépendent expression of IgH regulatory elements in stable B cell transfectants. Immunol Lett. 89:119.
41. Chung, J. H., M. Whiteley, and G. Felsenfeld. 1993. A 5' element of the chicken beta-globin domain serves as an insulator in human erythroid cells and protects against position effect in Drosophila. Cell 74:505.
42. Tajima, S., K. Shinohara, M. Fukumoto, R. Zaitsu, J. Miyagawa, S. Hino, J. Fan, K. Akasaka, and M. Matsuoka. 2006. Ars Insulator Identified in Sea Urchin Possesses an Activity to Ensure the Transgene Expression in Mouse Cells. J Biochem (Tokyo) 139:705.
43. T. Benton, T. Chen, M. McEntee, B. Fox, D. King, R. Crombie, T. C. Thomas, and C. Bebbington. The use of UCOE vectors in combination with a preadapted sérum free, suspension cell line allows for rapid production of large quantities of protein. Cytotechnology 38 (1-3):43-46, 2006.
44. Fukushige, S., and B. Sauer. 1992. Genomic targeting with a positive-selection lox integration vector allows highly reproducible gene expression in mammalian cells. Proc Natl. Acad. Sci U. S. A. 89:7905.
45. Kito, M., S. Itami, Y. Fukano, K. Yamana, and T. Shibui. 2002. Construction of engineered CHO strains for high-level production of recombinant proteins. Appl Microbiol. Biotechnol 60:442.
46. H. P. Fell, S. Yarnold, I. Hellstrom, K. E. Hellstrom, and K. R. Folger. Homologous recombination in hybridoma cells: heavy chain chimeric antibody produced by gene targeting. Proc Natl.Acad.Sci U.S A 86 (21):8507-8511, 1989.
47. D. A. Sorrell and A. F. Kolb. Targeted modification of mammalian genomes. Biotechnol Adv 23 (7-8):431-469, 2005.
48. Morrow, B., and R. Kucherlapati. 1993. Gene targeting in mammalian cells by homologous recombination. Curr Opin Biotechnol 4:577.
49. Choulika, A., A. Perrin, B. Dujon, and J. F. Nicolas. 1995. Induction of homologous recombination in mammalian chromosomes by using the I-SceI system of Saccharomyces cerevisiae. Mol. Cell. Biol. 15:1968.
50. Kolb, A. F. 2002. Genome engineering using site-specific recombinases. Cloning Stem Cells 4:65.
51. Sauer, B. 1987. Functional expression of the cre-lox site-specific recombination system in the yeast Saccharomyces cerevisiae. Mol Cell Biol 7:2087.
52. Sauer, B., and N. Henderson. 1990. Targeted insertion of exogenous DNA into the eukaryotic genome by the Cre recombinase. New. Biol 2:441.
53. Golic, K. G., and S. Lindquist. 1989. The FLP recombinase of yeast catalyzes site-specific recombination in the Drosophila genome. Cell 59:499.
54. O'Gorman, S., D. T. Fox, and G. M. Wahl. 1991. Recombinase-mediated gene activation and site-specific integration in mammalian cells. Science 251:1351.
55. Buchholz, F., P. O. Angrand, and A. F. Stewart. 1998. Improved properties of FLP recombinase evolved by cycling mutagenesis. Nat Biotechnol 16:657.
56. Xu, Z. L., H. Mizuguchi, A. Ishii-Watabe, E. Uchida, T. Mayumi, and T. Hayakawa. 2001. Optimization of transcriptional regulatory elements for constructing plasmid vectors. Gene 272:149.
57. Xu, Z. L., H. Mizuguchi, A. Ishii-Watabe, E. Uchida, T. Mayumi, and T. Hayakawa. 2002. Strength evaluation of transcriptional regulatory elements for transgene expression by adenovirus vector. J Control Release. 81:155.
58. Pfarr, D. S., L. A. Rieser, R. P. Woychik, F. M. Rottman, M. Rosenberg, and M. E. Reff. 1986. Differential effects of polyadenylation regions on gene expression in mammalian cells. DNA 5:115.
59. Petitclerc, D., J. Attal, M. C. Theron, M. Bearzotti, P. Bolifraud, G. Kann, M. G. Stinnakre, H. Pointu, C. Puissant, and L. M. Houdebine. 1995. The effect of various introns and transcription terminators on the efficiency of expression vectors in various cultured cell lines and in the mammary gland of transgenic mice. J Biotechnol 40:169.
60. Kim, D., J. D. Kim, K. Baek, Y. Yoon, and J. Yoon. 2003. Improved mammalian expression systems by manipulating transcriptional termination regions. Biotechnol Prog 19:1620.
61. Edmonds, M. 2002. A history of poly A sequences: from formation to factors to function. Prog. Nucleic Acid. Res Mol Biol 71:285.
62. Lewis, J. D., S. I. Gunderson, and I. W. Mattaj. 1995. The influence of 5' and 3' end structures on pre-mRNA metabolism. J Cell Sci Suppl 19:13.
63. Cramer, P., A. Srebrow, S. Kadener, S. Werbajh, M. M. de la, G. Melen, G. Nogues, and A. R. Kornblihtt. 2001. Coordination between transcription and pre-mRNA processing. FEBS. Lett. 498:179.
64. Carswell et al.. (1989), Molecular and Cellular Biology, Oct. 1989, p. 4248-4258.
65. Denome et al. (1988), Molecular and Cellular Biology, Nov. 1988, p. 4829-4839.
66. Gil A, Proudfoot NJ. (1987), Cell. 1987 May 8;49(3):399-406. Position-dependent sequence elements downstream of AAUAAA are required for efficient rabbit beta-globin mRNA 3' end formation.
67. Gimmi ER, Soprano KJ, Rosenberg M, Reff ME (1998), Nucleic Acids Res. 1988 Sep 26;16(18):8977-97. Deletions in the SV40 late polyadenylation region downstream of the AATAAA mediate similar effects on expression in various mammalian cell lines.
68. Schek N, Cooke C, Alwine JC (1992), Mol Cell Biol. 12 (12) :5386-93. Definition of the upstream efficiency element of the simian virus 40 late polyadenylation signal boy using in vitro analyses.
69. Levitt N, Briggs D, Gil A, Proudfoot NJ (1989), Genes Dev. 3(7):1019-25. Definition of an efficient synthetic poly(A) site.
70. Moreira A, Wollerton M, Monks J, Proudfoot NJ (1995) 14(15):3809-19 Upstream séquence elements enhance poly(A) site efficiency of the C2 complement gene and are phylogenetically conserved.
71. Edwalds-Gilbert G, Prescott J, Falck-Pedersen E. (1993), Mol Cell Biol. Jun;13(6):3472-80. 3' RNA processing efficiency plays a primary role in generating termination-competent RNA polymerase II elongation complexes.

## Revendications

1. Procédé d'obtention d'une lignée cellulaire fortement productrice d'une protéine d'intérêt comportant au moins une cellule, comprenant les étapes suivantes :
- l'intégration dans une zone de forte activité transcriptionnelle du génome de ladite cellule d'une molécule d'acide nucléique comprenant (i) deux séquences en tandem correspondant chacune à un site de reconnaissance d'une recombinase, entre lesquelles se trouvent un gène rapporteur, un gène codant une protéine d'intérêt, un gène de sélection et un gène de résistance à un antibiotique dépourvu de séquence de polyadénylation, et (ii) une séquence signal de polyadénylation de faible efficacité située en aval du deuxième site de reconnaissance d'une recombinase ;
- la sélection des cellules fortement productrices ayant intégré une copie unique de la molécule d'acide nucléique ;
- l'excision de la molécule d'acide nucléique par l'action de la recombinase ; et,
- la sélection des cellules formant des lignées cellulaires qui ont excisé la molécule d'acide nucléique en ne conservant qu'un site unique et intègre de reconnaissance d'une recombinase.

2. Procédé selon la revendication 1, dans lequel la molécule d'acide nucléique comporte en outre, entre les deux sites de reconnaissance d'une recombinase, un gène codant la thymidine kinase du virus Herpès simplex de type I (HSV1-TK).

3. Procédé selon les revendications 1 ou 2, dans lequel la série d'étapes comprend en outre une transfection de la lignée cellulaire sélectionnée avec un vecteur d'expression comprenant une séquence d'acide nucléique codant une protéine ou polypeptide d'intérêt et une séquence d'acide nucléique codant un site de reconnaissance de recombinase directement en aval d'une séquence d'acide nucléique codant un marqueur de sélection dépourvu de séquence de polyadénylation.

4. Procédé selon la revendication 3, dans lequel le marqueur de sélection est un gène de résistance à un antibiotique.

5. Lignée cellulaire obtenue selon le procédé de l'une quelconque des revendications 1 à 4.

6. Lignée cellulaire comprenant, intégré de manière stable dans son génome, un site unique de reconnaissance d'une recombinase dans une zone de forte activité transcriptionnelle du génome de ladite cellule et, directement en aval dudit site unique de reconnaissance d'une recombinase, une séquence d'acide nucléique codant un signal de polyadénylation de faible efficacité.

7. Lignée cellulaire selon la revendication 6, dans laquelle la cellule comprend, en outre :
- deux séquences d'acide nucléique codant chacune un site de reconnaissance d'une recombinase ;
- au moins une séquence d'acide nucléique codant une protéine d'intérêt entre les deux séquences codant chacune un site de reconnaissance d'une recombinase ; et
- au moins une séquence d'acide nucléique, codant un marqueur de sélection dépourvu de séquence de polyadénylation, entre les deux séquences codant chacune un site de reconnaissance d'une recombinase, ladite séquence codant pour un marqueur de sélection étant située directement en amont de la séquence d'acide nucléique codant ledit signal de polyadénylation de faible efficacité.

8. Lignée cellulaire selon la revendication 7, dans laquelle le marqueur de sélection est un gène de résistance à un antibiotique.

9. Lignée cellulaire, dans laquelle l'ensemble des séquences des revendications 7 et 8 sont intégrées ensemble en une seule copie.

10. Lignée cellulaire, dans laquelle la cellule possède un seul site de reconnaissance intègre de la recombinase et, en aval dudit site unique de reconnaissance d'une recombinase, une séquence d'acide nucléique codant un signal de polyadénylation de faible efficacité.

11. Lignée cellulaire selon la revendication 10, laquelle comprend une séquence d'acide nucléique codant un site de reconnaissance de recombinase en aval d'une séquence d'acide nucléique codant une protéine d'intérêt et une séquence d'acide nucléique codant un marqueur de sélection dépourvu de séquence de polyadénylation.

12. Lignée cellulaire selon la revendication 11, dans laquelle le marqueur de sélection est un gène de résistance à un antibiotique.

13. Lignée cellulaire, identifiée par la référence YGM-1/10G10, et déposée sous le numéro de dépôt CNCM 1-3704.

14. Lignée cellulaire, identifiée par la référence YGM-2/3G5, et déposée sous le numéro de dépôt CNCM I-3885.

15. Procédé de production d'au moins une protéine ou polypeptide d'intérêt, **caractérisé en ce qu'**on met en culture une lignée cellulaire selon l'une quelconque des revendications 6 à 14, de manière à exprimer ladite protéine d'intérêt, suivi d'au moins une étape de récupération de ladite protéine d'intérêt.

16. Vecteur comprenant la séquence SEQ ID NO : 2.

## Patentansprüche

1. Verfahren zum Erhalten einer hochproduktiven Zelllinie eines interessierenden Proteins, die mindestens eine Zelle umfasst, das die folgenden Schritte umfasst:
- Integrieren, in eine Zone mit hoher Transkriptionsaktivität des Genoms der Zelle, eines Nukleinsäuremoleküls, umfassend (i) zwei Tandemsequenzen, die jeweils einer Recombinase-Erkennungsstelle entsprechen, zwischen denen sich ein Reportergen, ein Gen, das ein interessierendes Protein kodiert, ein Selektionsgen und ein Antibiotikaresistenzgen befinden, ohne Polyadenylierungssequenz, und (ii) eine Polyadenylierungssignalsequenz mit geringer Wirksamkeit, die stromabwärts der zweiten Recombinase-Erkennungsstelle liegt;
- Auswählen der hochproduktiven Zellen, die eine Einzelkopie des Nukleinsäuremoleküls integriert haben;
- Exzidieren des Nukleinsäuremoleküls durch die Wirkung der Recombinase; und
- Auswählen der Zellen, die Zelllinien bilden, bei denen das Nukleinsäuremolekül exzidiert wurde und die nur eine einzige und vollständige Recombinase-Erkennungsstelle konservieren.

2. Verfahren nach Anspruch 1, wobei das Nukleinsäuremolekül ferner, zwischen den beiden Recombinase-Erkennungsstellen, ein Gen umfasst, das die Thymidinkinase des Herpes-Simplex-Virus, Typ I (HSV1-TK) kodiert.

3. Verfahren nach den Ansprüchen 1 oder 2, wobei die Abfolge von Schritten ferner eine Transfektion der ausgewählten Zelllinie mit einem Expressionsvektor umfasst, der eine Nukleinsäuresequenz, die ein interessierendes Protein oder Polypeptid kodiert, und eine Nukleinsäuresequenz umfasst, die eine Recombinase-Erkennungsstelle direkt stromabwärts einer Nukleinsäuresequenz kodiert, die einen Selektionsmarker ohne Polyadenylierungssequenz kodiert.

4. Verfahren nach Anspruch 3, wobei der Selektionsmarker ein Antibiotikaresistenzgen ist.

5. Zelllinie, die gemäß dem Verfahren nach einem der Ansprüche 1 bis 4 erhalten wurde.

6. Zelllinie, die, stabil in ihr Genom integriert, eine einzige Recombinase-Erkennungsstelle in einer Zone mit hoher Transkriptionsaktivität des Genoms der Zelle und, direkt stromabwärts dieser einzigen Recombinase-Erkennungsstelle, eine Nukleinsäuresequenz, die ein Polyadenylierungssignal mit geringer Wirksamkeit kodiert, umfasst.

7. Zelllinie nach Anspruch 6, wobei die Zelle ferner umfasst:
- zwei Nukleinsäuresequenzen, die jeweils eine Recombinase-Erkennungsstelle kodieren;
- mindestens eine Nukleinsäuresequenz, die ein interessierendes Protein zwischen den zwei Sequenzen kodiert, die jeweils eine Recombinase-Erkennungsstelle kodieren; und
- mindestens eine Nukleinsäuresequenz, die einen Selektionsmarker kodiert, ohne Polyadenylierungssequenz, zwischen den beiden Sequenzen, die jeweils eine Recombinase-Erkennungsstelle kodieren, wobei die Sequenz, die für einen Selektionsmarker kodiert, direkt stromaufwärts der Nukleinsäuresequenz liegt, die das Polyadenylierungssignal mit geringer Wirksamkeit kodiert.

8. Zelllinie nach Anspruch 7, wobei der Selektionsmarker ein Antibiotikaresistenzgen ist.

9. Zelllinie, wobei die Gesamtheit der Sequenzen der Ansprüche 7 und 8 zusammen in einer Einzelkopie integriert sind.

10. Zelllinie, wobei die Zelle eine einzige integrierte Recombinase-Erkennungsstelle aufweist und stromabwärts dieser einzigen Recombinase-Erkennungsstelle eine Nukleinsäuresequenz, die für ein Polyadenylierungssignal mit geringer Wirksamkeit kodiert.

11. Zelllinie nach Anspruch 10, die eine Nukleinsäuresequenz umfasst, die eine Recombinase-Erkennungsstelle stromabwärts einer Nukleinsäuresequenz kodiert, die ein interessierendes Protein kodiert, und eine Nukleinsäuresequenz, die einen Selektionsmarker ohne Polyadenylierungssequenz kodiert.

12. Zelllinie nach Anspruch 11, wobei der Selektionsmarker ein Antibiotikaresistenzgen ist.

13. Zelllinie, die durch die Referenz YGM-1/10G10 identifiziert und unter der Hinterlegungsnummer CNCM 1-3704 hinterlegt ist.

14. Zelllinie, die durch die Referenz YGM-2/3G5 identifiziert und unter der Hinterlegungsnummer CNCM 1-3885 hinterlegt ist.

15. Verfahren zur Herstellung mindestens eines interessierenden Proteins oder Polypeptids, **dadurch gekennzeichnet, dass** eine Zelllinie nach einem der Ansprüche 6 bis 14 kultiviert wird, um das interessierende Protein zu exprimieren, gefolgt von mindestens einem Schritt zur Gewinnung des interessierenden Proteins.

16. Vektor, umfassend die Sequenz SEQ ID NO: 2.

## Claims

1. Process to generate a cell line highly productive for a protein of interest comprising at least one cell, including the following steps:
- integration into a highly transcriptionally-active region of the genome of said cell of a nucleic acid sequence comprising (i) two sequences in tandem corresponding to a recombinase recognition site, between which there is a reporter gene, a gene encoding a protein similar to the protein of interest, a selector gene and a gene conferring resistance to an antibiotic, but no polyadenylation sequence; and (ii) a weakly active polyadenylation sequence located downstream of the second recombinase recognition site;
- the selection of highly productive cells carrying a single copy of the nucleic acid sequence;
- excision of the nucleic acid sequence by means of the recombinase, and
- the selection of cells to make cell lines which have lost the nucleic acid sequence, keeping only one intact, unique integrated recombinase recognition site.

2. Process according to claim 1, in which the nucleic acid sequence further contains, between the two recombinase recognition sites , a gene encoding the thymidine kinase of Type 1 Herpes simplex virus (HSV1-TK).

3. Process according to claim 1 or 2, in which the series of steps also includes transfection of the selected cell line with an expression vector carrying a nucleic acid sequence encoding a protein or polypeptide of interest and a nucleic acid sequence encoding a recombinase recognition site directly downstream of a nucleic acid sequence encoding a selection marker without any polyadenylation sequence.

4. Process according to claim 3, in which the selection marker is a gene conferring resistance to an antibiotic.

5. Cell line generated according to the process of any of claims 1 to 4.

6. Cell line carrying, stably integrated into its genome, a unique recombinase recognition site in a highly transcriptionally active region of the genome of said cell and directly downstream of said unique recombinase recognition site, a nucleic acid sequence encoding a weakly active polyadenylation sequence.

7. Cell line according to claim 6, in which the cell further comprises
- two nucleic acid sequences each encoding a recombinase recognition site;
- at least one nucleic acid sequence encoding a protein of interest between the two sequences each encoding a recombinase recognition site;
- at least one nucleic acid sequence, encoding a selection marker lacking polyadenylation sequence, located between the two sequences each coding a recombinase recognition site, this sequence coding for a selection marker being located directly upstream of the nucleic acid sequence encoding said weakly active polyadenylation sequence.

8. Cell line according to claim 7, in which the selection marker is a gene conferring resistance to an antibiotic.

9. Cell line, in which all the sequences of claims 7 and 8 are integrated together in one single copy.

10. Cell line in which the cell is carrying a unique recombinase recognition site and downstream of said unique recombinase recognition site, a nucleic acid sequence encoding a weakly active polyadenylation sequence.

11. Cell line according to claim 10, which comprises a nucleic acid sequence encoding a recombinase recognition site downstream a nucleic acid sequence encoding a protein of interest and a nucleic acid sequence encoding a selection marker lacking polyadenylation sequence.

12. Cell line according to claim 11, in which the selection marker is a gene conferring resistance to an antibiotic.

13. Cell line identified by the reference YGM-1/10G10 and registered under Application Number CNCM 1-3704.

14. Cell line identified by the reference YGM-2/3G5 registered under Application Number CNCM 1-3885.

15. Process for producing at least one protein or polypeptide of interest, **characterised in that** a cell line according to any of claims 6 to 14, is cultured in such a way that said protein of interest is expressed, followed by at least one step in which said protein of interest is harvested.

16. A vector comprising the sequence SEQ ID NO: 2.


### SEQUENCE LISTING

<110> LFB Biotechnologies SASU
<120> Lignée cellulaire à forte activité transcriptionnelle pour la production de protéines, notamment thérapeutiques
<130> Lignee a forte activite transcriptionnelle
<140> PCT/FR2007
   <141> 2007-12-20
<160> 16
<170> PatentIn version 3.3
<210> 1
   <211> 4419
   <212> DNA
   <213> Artificial
<220>
   <223> Isolated nucleic acid molecule
<220>
   <221> misc_feature
   <222> (140)..(140)
   <223> n is a, c, g, or t
<220>
   <221> misc_feature
   <222> (160)..(160)
   <223> n is a, c, g, or t
<400> 1
   tggaaacaga aactaaatag agacatagag aaaatgaaca gaagttatga accaaatgga    60
   tttaaccggt atttatacaa catttcatct taaaacaaaa gaatatacct tcttctcagc   120
   acctcatagc ctcttctacn aaaccatata gtcggtcacn aaacaagcct caacagatac   180
   aagaagataa aaataatccc atgcatacta tcagatcacc atggactaac tctggtcttc   240
   aataacaaca aaaacaatgg aagtcgaaga acactctatt caatgataac ttggtcaagg   300
   aagagatgaa aaaggaaatt aaagcctttt tagaatttca tgaaaatgaa ggcacaacat   360
   acccaaactt atgagacaca aggaaagcag tgctaagagg aaaactcata gctctgagtg   420
   cctccaaaaa gaaacaagag agagtgtata ctagcagctt gagagcacac ctgaaagctc   480
   tagaacaaaa agaagcaaat acacccaaga ggagaagatc aaatgcaggg ctgaaatcaa   540
   ccaagtagaa acaaaagaac tgtaaaaaga accaacaaaa ccaggagttg gttggttgag   600
   aaaatcaaaa agataaataa acccttaatc agaataacca gagggcacag aaacagtatc   660
   caaattaaca aaataagaaa tggaaaggga gacgtaaaac agaatccgag gaaataaaaa   720
   aaaattgatc ctattacaaa agtctatatt caacaaagct ggaaaatctg gatgaaatag   780
   acaattttct agatagatac cagataccaa agttaaatca ggaccagata aatcatctaa   840
   acagtcccat aactcctaaa gaaatagaag cagttattaa aattctcccg accaaaaaaa   900
   aaaaaaaaaa aaagcccagg attggatggg tttagtggag aattctatca gaccttcatc   960
   aaagacctaa taccaatact gtccaaacta ttccttgcaa cactgaatat tccgcataca   1020
   ctattcacag aaaagcatct tacggatggc atgacagtaa gagaattatg cagtgctgcc   1080
   ataaccatga gtgataacac tgcggccaac ttacttctga caacgatcgg aggaccgaag   1140
   gagctaaccg cttttttgca caacatgggg gatcatgtaa ctcgccttga tcgttgggaa   1200
   ccggagctga atgaagccat accaaacgac gagcgtgaca ccacgatgcc tgtagcaatg   1260
   gcaacaacgt tgcgcaaact attaactggc gaactactta ctctagcttc ccggcaacaa   1320
   ttaatagact ggatggaggc ggataaagtt gcaggaccac ttctgcgctc ggcccttccg   1380
   gctggctggt ttattgctga taaatctgga gccggtgagc gtgggtctcg cggtatcatt   1440
   gcagcactgg ggccagatgg taagccctcc cgtatcgtag ttatctacac gacggggagt   1500
   caggcaacta tggatgaacg aaatagacag atcgctgaga taggtgcctc actgattaag   1560
   cattggtaac tgtcagacca agtttactca tatatacttt agattgattt aaaacttcat   1620
   ttttaattta aaaggatcta ggtgaagatc ctttttgata atctcatgac caaaatccct   1680
   taacgtgagt tttcgttcca ctgagcgtca gaccccgtag aaaagatcaa aggatcttct   1740
   tgagatcctt tttttctgcg cgtaatctgc tgcttgcaaa caaaaaaacc accgctacca   1800
   gcggtggttt gtttgccgga tcaagagcta ccaactcttt ttccgaaggt aactggcttc   1860
   agcagagcgc agataccaaa tactgtcctt ctagtgtagc cgtagttagg ccaccacttc   1920
   aagaactctg tagcaccgcc tacatacctc gctctgctaa tcctgttacc agtggctgct   1980
   gccagtggcg ataagtcgtg tcttaccggg ttggactcaa gacgatagtt accggataag   2040
   gcgcagcggt cgggctgaac ggggggttcg tgcacacagc ccagcttgga gcgaacgacc   2100
   tacaccgaac tgagatacct acagcgtgag ctatgagaaa gcgccacgct tcccgaaggg   2160
   agaaaggcgg acaggtatcc ggtaagcggc agggtcggaa caggagagcg cacgagggag   2220
   cttccagggg gaaacgcctg gtatctttat agtcctgtcg ggtttcgcca cctctgactt   2280
   gagcgtcgat ttttgtgatg ctcgtcaggg gggcggagcc tatggaaaaa cgccagcaac   2340
   gcggcctttt tacggttcct ggccttttgc tggccttttg ctcacatggc tcgacagatc   2400
   catgttcttt cctgcgttat cccctgattc tgtggataac cgtattaccg cctttgagtg   2460
   agctgatacc gctcgccgca gccgaacgac cgagcgcagc gagtcagtga gcgaggaagc   2520
   ggaagagcgc ctgatgcggt attttctcct tacgcatctg tgcggtattt cacaccgcat   2580
   atggtgcact ctcagtacaa tctgctctga tgccgcatag ttaagccaga gaagtaccta   2640
   ttccgaagtt cctattctct agaaagtata ggaacttctc atgttctttc ctgcgttatc   2700
   ccctgattct gtggataacc gtattaccgc ctttgagtga gctgataccg ctcgccgcag   2760
   ccgaacgacc gagcgcagcg agtcagtgag cgaggaagcg gaagagcgcc tgatgcggta   2820
   ttttctcctt acgcatctgt gcggtatttc acaccgcata tggtgcactc tcagtacaat   2880
   ctgctctgat gccgcatagt taagccagct agaggatcat aatcagccat accacatttg   2940
   tagaggtttt acttgcttta aaaaacctcc cacacctccc cctgaacctg aaacataaaa   3000
   tgaatgcaat tgttgttgtt aacttgttta ttgcagctta taatggttac aaataaagca    3060
   atagcatcac aaatttcaca aataaagcat ttttttcact gcattctagt tgtggtttgt    3120
   ccaaactcat caatgtatct tatcatgtct ggatccccag gaagctcctc tgtgtcctca    3180
   taaaccctaa cctcctctac ttgagaggac attccaatca taggctgccc atccaccctc    3240
   tgtgtcctcc tgttaattag gtcacttaac aaaaaggaaa ttgggtaggg gtttttcaca    3300
   gaccgctttc taagggtaat tttaaaatat ctgggaagtc ccttccactg ctgtgttcca    3360
   gaagtgttgg taaacagccc acaaatgtca acagcagaaa catacaagct gtcagctttg    3420
   cacaagggcc caacaccctg ctcatcaaga agcactgtgg ttgctgtgtt agtaatgtgc    3480
   aaaacaggag gcacattttc cccacctgtg taggttccaa aatatctagt gttttcattt    3540
   ttacttggat caggaaccca gcactccact ggataagcat tatccttatc caaaacagcc    3600
   ttgtggtcag tgttcatctg ctgactgtca actgtagcat tttttggggt tacagtttga    3660
   gcaggatatt tggtcctgta gtttgctaac acaccctgca gctccaaagg ttccccacca    3720
   acagcaaaaa aatgaaaatt tgacccttga atgggttttc cagcaccatt ttcatgagtt    3780
   ttttgtgtcc ctgaatgcaa gtttaacata gcagttaccc caataacctc agttttaaca    3840
   gtaacagctt cccacatcaa aatatttcca caggttaagt cctcatttaa attaggcaaa    3900
   ggaattcttg aagacgaaag ggcctcgtga tacgcctatt tttataggtt aatgtcatga    3960
   taataatggt ttcttagacg tcaggtggca cttttcgggg aaatgtgcgc ggaaccccta    4020
   tttgtttatt tttctaaata cattcaaata tgtatccgct catgagacaa taaccctgat    4080
   aaatgcttca ataatattga aaaaggaaga gtatgagtat tcaacatttc cgtgtcgccc    4140
   ttattccctt ttttgcggca ttttgccttc ctgtttttgc tcacccagaa acgctggtga    4200
   aagtaaaaga tgctgaagat cagttgggtg cacgagtggg ttacatcgaa ctggatctca    4260
   acagcggtaa gatccttgag agttttcgcc ccgaagaacg ttttccaatg atgagcactt    4320
   ttaaagttct gctatgtggc gcggtattat cccgtgttga cgccgggcaa gagcaactcg    4380
   gtcgccgcat acactattct cagaatgact tggttgagt                           4419
<210> 2
   <211> 14987
   <212> DNA
   <213> Artificial
<220>
   <223> vector
<400> 2
   gaagtaccta ttccgaagtt cctattctct agaaagtata ggaacttcta cagagatccg     60
   cccgccccgt tgacgcaaat gggcggtagg cgtgtacggt gggaggtcta tataagcaga    120
   gctcgtttag tgaaccgtca gatcactaga agctttattg cggtagttta tcacagttaa    180
   attgctaacg cagtcagtgc ttctgacaca acagtctcga acttaagctg cagtgactct    240
   cttaaggtag ccttgcagaa gttggtcgtg aggcactggg caggtaagta tcaaggttac    300
   aagacaggtt taaggagacc aatagaaact gggcttgtcg agacagagaa gactcttgcg 360
   tttctgatag gcacctattg gtcttactga catccacttt gcctttctct ccacaggtgt 420
   ccactcccag ttcaattaca gctcttaagg ctagagtggg atccaccggt cgccaccatg 480
   gagagcgacg agagcggcct gcccgccatg gagatcgagt gccgcatcac cggcaccctg 540
   aacggcgtgg agttcgagct ggtgggcggc ggagagggca cccccgagca gggccgcatg 600
   accaacaaga tgaagagcac caaaggcgcc ctgaccttca gcccctacct gctgagccac 660
   gtgatgggct acggcttcta ccacttcggc acctacccca gcggctacga gaaccccttc 720
   ctgcacgcca tcaacaacgg cggctacacc aacacccgca tcgagaagta cgaggacggc 780
   ggcgtgctgc acgtgagctt cagctaccgc tacgaggccg gccgcgtgat cggcgacttc 840
   aaggtgatgg gcaccggctt ccccgaggac agcgtgatct tcaccgacaa gatcatccgc 900
   agcaacgcca ccgtggagca cctgcacccc atgggcgata acgatctgga tggcagcttc 960
   acccgcacct tcagcctgcg cgacggcggc tactacagct ccgtggtgga cagccacatg 1020
   cacttcaaga gcgccatcca ccccagcatc ctgcagaacg gggggcccat gttcgccttc 1080
   cgccgcgtgg aggaggatca cagcaacacc gagctgggca tcgtggagta ccagcacgcc 1140
   ttcaagaccc cggatgcaga tgccggtgaa gaataaagcg gcctagggat aacagggtaa 1200
   tggccgcgac tctagatcat aatcagccat accacatttg tagaggtttt acttgcttta 1260
   aaaaacctcc cacacctccc cctgaacctg aaacataaaa tgaatgcaat tgttgttgtt 1320
   aacttgttta ttgcagctta taatggttac aaataaagca atagcatcac aaatttcaca 1380
   aataaagcat ttttttcact gcattctagt tgtggtttgt ccaaactcat caatgtatct 1440
   taaggcgtaa attgtaagcg ttaatatttt gttaaaattc gcgttaaatt tttgttaaat 1500
   cagctcattt tttaaccaat aggccgaaat cggcaaaatc ccttataaat caaaagaata 1560
   gaccgagata gggttgagtg ttgttccagt ttggaacaag agtccactat taaagaacgt 1620
   ggactccaac gtcaaagggc gaaaaaccgt ctatcagggc gatggcccac gatcttagat 1680
   atccggacgt ggatctcccg atcccctatg gtgcactctc agtacaatct gctctgatgc 1740
   cgcatagtta agccagtatc tgctccctgc ttgtgtgttg gaggtcgctg agtagtgcgc 1800
   gagcaaaatt taagctacaa caaggcaagg cttgaccgac aattgcatga agaatctgct 1860
   tagggttagg cgttttgcgc tgcttcgcga tgtacgggcc agatatacgc gtatctgagg 1920
   ggactagggt gtgtttaggc gaaaagcggg gcttcggttg tacgcggtta ggagtcccct 1980
   caggatatag tagtttcgct tttgcatagg gagggggaaa tgtagtctta tgcaatactc 2040
   ttgtagtctt gcaacatggt aacgatgagt tagcaacatg ccttacaagg agagaaaaag 2100
   caccgtgcat gccgattggt ggaagtaagg tggtacgatc gtgccttatt aggaaggcaa 2160
   cagacgggtc tgacatggat tggacgaacc actgaattcc gcattgcaga gatattgtat 2220
   ttaagtgcct agctcgatac aataaacgcc atttgaccat tcaccacatt ggtgtgcacc 2280
   tccaagcttg gtaccgagct cggatccact agtgcagaag ttggtcgtga ggcactgggc 2340
   aggtaagtat caaggttaca agacaggttt aaggagacca atagaaactg ggcttgtcga    2400
   gacagagaag actcttgcgt ttctgatagg cacctattgg tcttactgac atccactttg    2460
   cctttctctc cacaggtgtc cactcccagt tcaattacag ctcttgctag tgccgccacc    2520
   atggagtttg ggctgagctg ggttttcctc gttgctcttt taagaggtgt ccagtgtcag    2580
   gtgcagctgg tggagtctgg gggaggcgtg gtccagcctg ggaggtccct gagactctcc    2640
   tgtacagcct ctggattcac cttcaaaaac tatgctatgc attgggtccg ccaggctcca    2700
   gccaaggggc tggagtgggt ggcaactata tcatatgatg gaaggaatat acaatatgca    2760
   gactccgtga agggccgatt caccttctcc agagacaatt ctcaggacac cctgtatctg    2820
   caactgaaca gcctcagacc ggaggacacg gctgtgtatt actgtgcgag acccgtaaga    2880
   agccgatggc tgcaattagg tcttgaagat gcttttcata tctggggcca ggggacaatg    2940
   gtcaccgtct cttcagcctc caccaagggc ccatcggtct tccccctggc accctcctcc    3000
   aagagcacct ctgggggcac agcggccctg ggctgcctgg tcaaggacta cttccccgaa    3060
   ccggtgacgg tgtcgtggaa ctcaggcgcc ctgaccagcg gcgtgcacac cttcccggct    3120
   gtcctacagt cctcaggact ctactccctc agcagcgtgg tgaccgtgcc ctccagcagc    3180
   ttgggcaccc agacctacat ctgcaacgtg aatcacaagc ccagcaacac caaggtggac    3240
   aagaaagttg agcccaaatc ttgtgacaaa actcacacat gcccaccgtg cccagcacct    3300
   gaactcctgg ggggaccgtc agtcttcctc ttccccccaa aacccaagga caccctcatg    3360
   atctcccgga cccctgaggt cacatgcgtg gtggtggacg tgagccacga agaccctgag    3420
   gtcaagttca actggtacgt ggacggcgtg gaggtgcata atgccaagac aaagccgcgg    3480
   gaggagcagt acaacagcac gtaccgtgtg gtcagcgtcc tcaccgtcct gcaccaggac    3540
   tggctgaatg gcaaggagta caagtgcaag gtctccaaca aagccctccc agcccccatc    3600
   gagaaaacca tctccaaagc caaagggcag ccccgagaac cacaggtgta caccctgccc    3660
   ccatcccggg atgagctgac caagaaccag gtcagcctga cctgcctggt caaaggcttc    3720
   tatcccagcg acatcgccgt ggagtgggag agcaatgggc agccggagaa caactacaag    3780
   accacgcctc ccgtgctgga ctccgacggc tccttcttcc tctacagcaa gctcaccgtg    3840
   gacaagagca ggtggcagca ggggaacgtc ttctcatgct ccgtgatgca tgaggctctg    3900
   cacaaccact acacgcagaa gagcctctcc ctgtctccgg gtaaatgata gtctagagct    3960
   cgctgatcag cctcgactgt gccttctagt tgccagccat ctgttgtttg cccctccccc    4020
   gtgccttcct tgaccctgga aggtgccact cccactgtcc tttcctaata aaatgaggaa    4080
   attgcatcgc attgtctgag taggtgtcat tctattctgg ggggtggggt ggggcaggac    4140
   agcaaggggg aggattggga agacaatagc aggcatgctg gggatgcggt gggctctatg    4200
   gcttctgagg cggaaagaac cagctggggc tcgagatctc ccgatcccct atggtgcact    4260
   ctcagtacaa tctgctctga tgccgcatag ttaagccagt atctgctccc tgcttgtgtg    4320
   ttggaggtcg ctgagtagtg cgcgagcaaa atttaagcta caacaaggca aggcttgacc    4380
   gacaattgca tgaagaatct gcttagggtt aggcgttttg cgctgcttcg cgatgtacgg 4440
   gccagatata cgcgtatctg aggggactag ggtgtgttta ggcgaaaagc ggggcttcgg 4500
   ttgtacgcgg ttaggagtcc cctcaggata tagtagtttc gcttttgcat agggaggggg 4560
   aaatgtagtc ttatgcaata ctcttgtagt cttgcaacat ggtaacgatg agttagcaac 4620
   atgccttaca aggagagaaa aagcaccgtg catgccgatt ggtggaagta aggtggtacg 4680
   atcgtgcctt attaggaagg caacagacgg gtctgacatg gattggacga accactgaat 4740
   tccgcattgc agagatattg tatttaagtg cctagctcga tacaataaac gccatttgac 4800
   cattcaccac attggtgtgc acctccaagc ttggtaccga gctcggatcc actagtgcag 4860
   aagttggtcg tgaggcactg ggcaggtaag tatcaaggtt acaagacagg tttaaggaga 4920
   ccaatagaaa ctgggcttgt cgagacagag aagactcttg cgtttctgat aggcacctat 4980
   tggtcttact gacatccact ttgcctttct ctccacaggt gtccactccc agttcaatta 5040
   cagctcttgc tagtgccgcc accatgaggg tccccgctca gctcctgggg ctcctgctgc 5100
   tctggctccc aggtgccaga tgtgccatcc ggatgaccca gtctccatcc tcattctctg 5160
   catctacagg agacagagtc accatcactt gtcgggcgag ccaggatatt cggaactatg 5220
   tagcctggta tcagcaaaaa tcagggaaag cccctaaatt cctgatctat gctgcttcca 5280
   ctttgcaaag tggggtccca tcaaggttca gcggcagtgg atctgggaca gatttcactc 5340
   tcaccatcaa ctccctgcag tctgaagatt ttgcaactta ttactgtcaa caatattaca 5400
   attctcctcc gaccttcggc caagggacca gggtggaaat cacgcgaact gtggctgcac 5460
   catctgtctt catcttcccg ccatctgatg agcagttgaa atctggaact gcctctgttg 5520
   tgtgcctgct gaataacttc tatcccagag aggccaaagt acagtggaag gtggataacg 5580
   ccctccaatc gggtaactcc caggagagtg tcacagagca ggacagcaag gacagcacct 5640
   acagcctcag cagcaccctg acgctgagca aagcagacta cgagaaacac aaagtctacg 5700
   cctgcgaagt cacccatcag ggcctgagct cgcccgtcac aaagagcttc aacaggggag 5760
   agtgttgata gtctagagct cgctgatcag cctcgactgt gccttctagt tgccagccat 5820
   ctgttgtttg cccctccccc gtgccttcct tgaccctgga aggtgccact cccactgtcc 5880
   tttcctaata aaatgaggaa attgcatcgc attgtctgag taggtgtcat tctattctgg 5940
   ggggtggggt ggggcaggac agcaaggggg aggattggga agacaatagc aggcatgctg 6000
   gggatgcggt gggctctatg gcttctgagg cggaaagaac cagtcgaggg gggatcccca 6060
   cgcgccctgt agcggcgcat taagcgcggc gggtgtggtg gttacgcgca gcgtgaccgc 6120
   tacacttgcc agcgccctag cgcccgctcc tttcgctttc ttcccttcct ttctcgccac 6180
   gttcgccggc tttccccgtc aagctctaaa tcggggcatc cctttagggt tccgatttag 6240
   tgctttacgg cacctcgacc ccaaaaaact tgattagggt gatggttcac gtatagggat 6300
   aacagggtaa tgtagtgggc catcgccctg atagacggtt tttcgccctt tgacgttgga 6360
   gtccacgttc tttaatagtg gactcttgtt ccaaactgga acaacactca accctatctc 6420
   ggtctattct tttgatttat aagggatttt ggggatttcg gcctattggt taaaaaatga 6480
   gctgatttaa caaaaattta acgcgaattt taacaaaata ttaacgttta caatttaaat 6540
   atttgcttat acaatcttcc tgtttttggg gcttttctga ttatcaaccg gggtgggtac 6600
   cgagctcgaa ttctgtggaa tgtgtgtcag ttagggtgtg gaaagtcccc aggctcccca 6660
   ggcaggcaga agtatgcaaa gcatgcatct caattagtca gcaaccaggt gtggaaagtc 6720
   cccaggctcc ccagcaggca gaagtatgca aagcatgcat ctcaattagt cagcaaccat 6780
   agtcccgccc ctaactccgc ccatcccgcc cctaactccg cccagttccg cccattctcc 6840
   gccccatggc tgactaattt tttttattta tgcagaggcc gaggccgcct cggcctctga 6900
   gctattccag aagtagtgag gaggcttttt tggaggccta ggcttttgca aaaagctccc 6960
   gggagcttgg atatccattt tcggatctga tcaagagaca ggatgaggat cgtttcgcat 7020
   gattgaacaa gatggattgc acgcaggttc tccggccgct tgggtggaga ggctattcgg 7080
   ctatgactgg gcacaacaga caatcggctg ctctgatgcc gccgtgttcc ggctgtcagc 7140
   gcaggggcgc ccggttcttt ttgtcaagac cgacctgtcc ggtgccctga atgaactgca 7200
   ggacgaggca gcgcggctat cgtggctggc cacgacgggc gttccttgcg cagctgtgct 7260
   cgacgttgtc actgaagcgg gaagggactg gctgctattg ggcgaagtgc cggggcagga 7320
   tctcctgtca tctcaccttg ctcctgccga gaaagtatcc atcatggctg atgcaatgcg 7380
   gcggctgcat acgcttgatc cggctacctg cccattcgac caccaagcga aacatcgcat 7440
   cgagcgagca cgtactcgga tggaagccgg tcttgtcgat caggatgatc tggacgaaga 7500
   gcatcagggg ctcgcgccag ccgaactgtt cgccaggctc aaggcgcgca tgcccgacgg 7560
   cgaggatctc gtcgtgaccc atggcgatgc ctgcttgccg aatatcatgg tggaaaatgg 7620
   ccgcttttct ggattcatcg actgtggccg gctgggtgtg gcggaccgct atcaggacat 7680
   agcgttggct acccgtgata ttgctgaaga gcttggcggc gaatgggctg accgcttcct 7740
   cgtgctttac ggtatcgccg ctcccgattc gcagcgcatc gccttctatc gccttcttga 7800
   cgagttcttc tgagcgggac tctggggttc gaaatgaccg accaagcgac gcccaacctg 7860
   ccatcacgag atttcgattc caccgccgcc ttctatgaaa ggttgggctt cggaatcgtt 7920
   ttccgggacg ccggctggat gatcctccag cgcggggatc tcatgctgga gttcttcgcc 7980
   caccccaact tgtttattgc agcttataat ggttacaaat aaagcaatag catcacaaat 8040
   ttcacaaata aagcattttt ttcactgcat tctagttgtg gtttgtccaa actcatcaat 8100
   gtatcttatc atgtctggat cccgtcgagt ttaaacgatt taaatactgg ggctcgactg 8160
   tggaatgtgt gtcagttagg gtgtggaaag tccccaggct ccccagcagg cagaagtatg 8220
   caaagcatgc atctcaatta gtcagcaacc aggtgtggaa agtccccagg ctccccagca 8280
   ggcagaagta tgcaaagcat gcatctcaat tagtcagcaa ccatagtccc gcccctaact 8340
   ccgcccatcc cgcccctaac tccgcccagt tccgcccatt ctccgcccca tggctgacta 8400
   atttttttta tttatgcaga ggccgaggcc gcctcggcct ctgagctatt ccagaagtag 8460
   tgaggaggct tttttggagg cctaggcttt tgcaaaaagc ttgggggggg ggacagctca 8520
   gggctgcgat ttcgcgccaa acttgacggc aatcctagcg tgaaggctgg taggatttta 8580
   tccccgctgc catcatggtt cgaccattga actgcatcgt cgccgtgtcc caaaatatgg 8640
   ggattggcaa gaacggagac ctaccctggc ctccgctcag gaacgagttc aagtatttcc 8700
   aaagaatgac cacaacctct tcagtggaag gtaaacagaa tctggtgatt atgggtagga 8760
   aaacctggtt ctccattcct gagaagaatc gacctttaaa ggacagaatt aatatagttc 8820
   tcagtagaga actcaaagaa ccaccacgag gagctcattt tcttgccaaa agtttggatg 8880
   atgccttaag acttattgaa caaccggaat tggcaagtaa agtagacatg gtttggatag 8940
   tcggaggcag ttctgtttac caggaagcca tgaatcaacc aggccacctc agactctttg 9000
   tgacaaggat catgcaggaa tttgaaagtg acacgttttt cccagaaatt gatttgggga 9060
   aatataaact tctcccagaa tacccaggcg tcctctctga ggtccaggag gaaaaaggca 9120
   tcaagtataa gtttgaagtc tacgagaaga aagactaaca ggaagatgct ttcaagttct 9180
   ctgctcccct cctaaagcta tgcattttta taagaccatg ggacttttgc tggctttaga 9240
   tcgatctttg tgaaggaacc ttacttctgt ggtgtgacat aattggacaa actacctaca 9300
   gagatttaaa gctctaaggt aaatataaaa tttttaagtg tataatgtgt taaactactg 9360
   attctaattg tttgtgtatt ttagattcca acctatggaa ctgatgaatg ggagcagtgg 9420
   tggaatgcct ttaatgagga aaacctgttt tgctcagaag aaatgccatc tagtgatgat 9480
   gaggctactg ctgactctca acattctact cctccaaaaa agaagagaaa ggtagaagac 9540
   cccaaggact ttccttcaga attgctaagt tttttgagtc atgctgtgtt tagtaataga 9600
   actcttgctt gctttgctat ttacaccaca aaggaaaaag ctgcactgct atacaagaaa 9660
   attatggaaa aatattctgt aacctttata agtaggcata acagttataa tcataacata 9720
   ctgttttttc ttactccaca caggcataga gtgtctgcta ttaataacta tgctcaaaaa 9780
   ttgtgtacct ttagcttttt aatttgtaaa ggggttaata aggaatattt gatgtatagt 9840
   gccttgacta gagatcataa tcagccatac cacatttgta gaggttttac ttgctttaaa 9900
   aaacctccca cacctccccc tgaacctgaa acataaaatg aatgcaattg ttgttgttaa 9960
   cttgtttatt gcagcttata atggttacaa ataaagcaat agcatcacaa atttcacaaa 10020
   taaagcattt ttttcactgc attctagttg tggtttgtcg acgcggccgc tgtggaatgt 10080
   gtgtcagtta gggtgtggaa agtccccagg ctccccagca ggcagaagta tgcaaagcat 10140
   gcatctcaat tagtcagcaa ccaggtgtgg aaagtcccca ggctccccag caggcagaag 10200
   tatgcaaagc atgcatctca attagtcagc aaccatagtc ccgcccctaa ctccgcccat 10260
   cccgccccta actccgccca gttccgccca ttctccgccc catggctgac taattttttt 10320
   tatttatgca gaggccgagg ccgcctcggc ctctgagcta ttccagaagt agtgaggagg 10380
   cttttttgga ggcctaggct tttgcaaaaa gctctgagat caccggcgaa ggagggccac 10440
   catggcctcg taccccggcc atcaacacgc gtctgcgttc gaccaggctg cgcgttctcg 10500
   cggccatagc aaccgacgta cggcgttgcg ccctcgccgg cagcaagaag ccacggaagt 10560
   ccgcccggag cagaaaatgc ccacgctact gcgggtttat atagacggtc cccacgggat 10620
   ggggaaaacc accaccacgc aactgctggt ggccctgggt tcgcgcgacg atatcgtcta 10680
   cgtacccgag ccgatgactt actggcgggt gctgggggct tccgagacaa tcgcgaacat 10740
   ctacaccaca caacaccgcc tcgaccaggg tgagatatcg gccggggacg cggcggtggt 10800
   aatgacaagc gcccagataa caatgggcat gccttatgcc gtgaccgacg ccgttctggc 10860
   tcctcatatc gggggggagg ctgggagctc acatgccccg cccccggccc tcaccctcat 10920
   cttcgaccgc catcccatcg ccgccctcct gtgctacccg gccgcgcggt accttatggg 10980
   cagcatgacc ccccaggccg tgctggcgtt cgtggccctc atcccgccga ccttgcccgg 11040
   caccaacatc gtgcttgggg cccttccgga ggacagacac atcgaccgcc tggccaaacg 11100
   ccagcgcccc ggcgagcggc tggacctggc tatgctggct gcgattcgcc gcgtttacgg 11160
   gctacttgcc aatacggtgc ggtatctgca gtgcggcggg tcgtggcggg aggactgggg 11220
   acagctttcg gggacggccg tgccgcccca gggtgccgag ccccagagca acgcgggccc 11280
   acgaccccat atcggggaca cgttatttac cctgtttcgg gcccccgagt tgctggcccc 11340
   caacggcgac ctgtataacg tgtttgcctg ggccttggac gtcttggcca aacgcctccg 11400
   ttccatgcac gtctttatcc tggattacga ccaatcgccc gccggctgcc gggacgccct 11460
   gctgcaactt acctccggga tggtccagac ccacgtcacc acccccggct ccataccgac 11520
   gatatgcgac ctggcgcgca cgtttgcccg ggagatgggg gaggctaact gagaatttcg 11580
   actgaagtac ctattccgaa gttcctattc tctagaaagt ataggaactt ctcatgttct 11640
   ttcctgcgtt atcccctgat tctgtggata accgtattac cgcctttgag tgagctgata 11700
   ccgctcgccg cagccgaacg accgagcgca gcgagtcagt gagcgaggaa gcggaagagc 11760
   gcctgatgcg gtattttctc cttacgcatc tgtgcggtat ttcacaccgc atatggtgca 11820
   ctctcagtac aatctgctct gatgccgcat agttaagcca gctagaggat cataatcagc 11880
   cataccacat ttgtagaggt tttacttgct ttaaaaaacc tcccacacct ccccctgaac 11940
   ctgaaacata aaatgaatgc aattgttgtt gttaacttgt ttattgcagc ttataatggt 12000
   tacaaataaa gcaatagcat cacaaatttc acaaataaag catttttttc actgcattct 12060
   agttgtggtt tgtccaaact catcaatgta tcttatcatg tctggatccc caggaagctc 12120
   ctctgtgtcc tcataaaccc taacctcctc tacttgagag gacattccaa tcataggctg 12180
   cccatccacc ctctgtgtcc tcctgttaat taggtcactt aacaaaaagg aaattgggta 12240
   ggggtttttc acagaccgct ttctaagggt aattttaaaa tatctgggaa gtcccttcca 12300
   ctgctgtgtt ccagaagtgt tggtaaacag cccacaaatg tcaacagcag aaacatacaa 12360
   gctgtcagct ttgcacaagg gcccaacacc ctgctcatca agaagcactg tggttgctgt 12420
   gttagtaatg tgcaaaacag gaggcacatt ttccccacct gtgtaggttc caaaatatct 12480
   agtgttttca tttttacttg gatcaggaac ccagcactcc actggataag cattatcctt 12540
   atccaaaaca gccttgtggt cagtgttcat ctgctgactg tcaactgtag cattttttgg 12600
   ggttacagtt tgagcaggat atttggtcct gtagtttgct aacacaccct gcagctccaa 12660
   aggttcccca ccaacagcaa aaaaatgaaa atttgaccct tgaatgggtt ttccagcacc 12720
   attttcatga gttttttgtg tccctgaatg caagtttaac atagcagtta ccccaataac 12780
   ctcagtttta acagtaacag cttcccacat caaaatattt ccacaggtta agtcctcatt 12840
   taaattaggc aaaggaattc ttgaagacga aagggcctcg tgatacgcct atttttatag 12900
   gttaatgtca tgataataat ggtttcttag acgtcaggtg gcacttttcg gggaaatgtg 12960
   cgcggaaccc ctatttgttt atttttctaa atacattcaa atatgtatcc gctcatgaga 13020
   caataaccct gataaatgct tcaataatat tgaaaaagga agagtatgag tattcaacat 13080
   ttccgtgtcg cccttattcc cttttttgcg gcattttgcc ttcctgtttt tgctcaccca 13140
   gaaacgctgg tgaaagtaaa agatgctgaa gatcagttgg gtgcacgagt gggttacatc 13200
   gaactggatc tcaacagcgg taagatcctt gagagttttc gccccgaaga acgttttcca 13260
   atgatgagca cttttaaagt tctgctatgt ggcgcggtat tatcccgtgt tgacgccggg 13320
   caagagcaac tcggtcgccg catacactat tctcagaatg acttggttga gtactcacca 13380
   gtcacagaaa agcatcttac ggatggcatg acagtaagag aattatgcag tgctgccata 13440
   accatgagtg ataacactgc ggccaactta cttctgacaa cgatcggagg accgaaggag 13500
   ctaaccgctt ttttgcacaa catgggggat catgtaactc gccttgatcg ttgggaaccg 13560
   gagctgaatg aagccatacc aaacgacgag cgtgacacca cgatgcctgt agcaatggca 13620
   acaacgttgc gcaaactatt aactggcgaa ctacttactc tagcttcccg gcaacaatta 13680
   atagactgga tggaggcgga taaagttgca ggaccacttc tgcgctcggc ccttccggct 13740
   ggctggttta ttgctgataa atctggagcc ggtgagcgtg ggtctcgcgg tatcattgca 13800
   gcactggggc cagatggtaa gccctcccgt atcgtagtta tctacacgac ggggagtcag 13860
   gcaactatgg atgaacgaaa tagacagatc gctgagatag gtgcctcact gattaagcat 13920
   tggtaactgt cagaccaagt ttactcatat atactttaga ttgatttaaa acttcatttt 13980
   taatttaaaa ggatctaggt gaagatcctt tttgataatc tcatgaccaa aatcccttaa 14040
   cgtgagtttt cgttccactg agcgtcagac cccgtagaaa agatcaaagg atcttcttga 14100
   gatccttttt ttctgcgcgt aatctgctgc ttgcaaacaa aaaaaccacc gctaccagcg 14160
   gtggtttgtt tgccggatca agagctacca actctttttc cgaaggtaac tggcttcagc 14220
   agagcgcaga taccaaatac tgtccttcta gtgtagccgt agttaggcca ccacttcaag 14280
   aactctgtag caccgcctac atacctcgct ctgctaatcc tgttaccagt ggctgctgcc 14340
   agtggcgata agtcgtgtct taccgggttg gactcaagac gatagttacc ggataaggcg 14400
   cagcggtcgg gctgaacggg gggttcgtgc acacagccca gcttggagcg aacgacctac 14460
   accgaactga gatacctaca gcgtgagcta tgagaaagcg ccacgcttcc cgaagggaga 14520
   aaggcggaca ggtatccggt aagcggcagg gtcggaacag gagagcgcac gagggagctt 14580
   ccagggggaa acgcctggta tctttatagt cctgtcgggt ttcgccacct ctgacttgag    14640
   cgtcgatttt tgtgatgctc gtcagggggg cggagcctat ggaaaaacgc cagcaacgcg    14700
   gcctttttac ggttcctggc cttttgctgg ccttttgctc acatggctcg acagatccat    14760
   gttctttcct gcgttatccc ctgattctgt ggataaccgt attaccgcct ttgagtgagc    14820
   tgataccgct cgccgcagcc gaacgaccga gcgcagcgag tcagtgagcg aggaagcgga    14880
   agagcgcctg atgcggtatt ttctccttac gcatctgtgc ggtatttcac accgcatatg    14940
   gtgcactctc agtacaatct gctctgatgc cgcatagtta agccaga                  14987
<210> 3
   <211> 5023
   <212> DNA
   <213> Artificial
<220>
   <223> vector
<400> 3
   gatctccagg gaccgtccct aaattctcac agacccaaat ccctgtagcc gccccacgac     60
   agcgcgagga gcatgcgccc agggctgagc gcgggtagat cagagcacac aagctcacag    120
   tccccggcgg tggggggagg ggcgcgctga gcgggggcca gggagctggc gcggggcaaa    180
   ctgggaaagt ggtgtcgtgt gctggctccg ccctcttccc gagggtgggg gagaacggta    240
   tataagtgcg gtagtcgcct tggacgttct ttttcgcaac gggtttgccg tcagaacgca    300
   ggtgagtggc gggtgtggct tccgcgggcc ccggagctgg agccctgctc tgagcgggcc    360
   gggctgatat gcgagtgtcg tccgcagggt ttagctgtga gcattcccac ttcgagtggc    420
   gggcggtgcg ggggtgagag tgcgaggcct agcggcaacc ccgtagcctc gcctcgtgtc    480
   cggcttgagg cctagcgtgg tgtccgccgc cgcgtgccac tccggccgca ctatgcgttt    540
   tttgtccttg ctgccctcga ttgccttcca gcagcatggg ctaacaaagg gagggtgtgg    600
   ggctcactct taaggagccc atgaagctta cgttggatag gaatggaagg gcaggagggg    660
   cgactggggc ccgcccgcct tcggagcaca tgtccgacgc cacctggatg gggcgaggcc    720
   tgtggctttc cgaagcaatc gggcgtgagt ttagcctacc tgggccatgt ggccctagca    780
   ctgggcacgg tctggcctgg cggtgccgcg ttcccttgcc tcccaacaag ggtgaggccg    840
   tcccgcccgg caccagttgc ttgcgcggaa agatggccgc tcccggggcc ctgttgcaag    900
   gagctcaaaa tggaggacgc ggcagcccgg tggagcgggc gggtgagtca cccacacaaa    960
   ggaagagggc cttgcccctc gccggccgct gcttcctgtg accccgtggt ctatcggccg   1020
   catagtcacc tcgggcttct cttgagcacc gctcgtcgcg gcggggggag gggatctaat   1080
   ggcgttggag tttgttcaca tttggtgggt ggagactagt caggccagcc tggcgctgga   1140
   agtcattctt ggaatttgcc cctttgagtt tggagcgagg ctaattctca agcctcttag   1200
   cggttcaaag gtattttcta aacccgtttc caggtgttgt gagctagccg ccaccatgcc   1260
   acaatttgat atattatgta aaacaccacc taaggtcctg gttcgtcagt ttgtggaaag   1320
   gtttgaaaga ccttcagggg aaaaaatagc atcatgtgct gctgaactaa cctatttatg 1380
   ttggatgatt actcataacg gaacagcaat caagagagcc acattcatga gctataatac 1440
   tatcataagc aattcgctga gtttcgatat tgtcaacaaa tcactccagt ttaaatacaa 1500
   gacgcaaaaa gcaacaattc tggaagcctc attaaagaaa ttaattcctg cttgggaatt 1560
   tacaattatt ccttacaatg gacaaaaaca tcaatctgat atcactgata ttgtaagtag 1620
   tttgcaatta cagttcgaat catcggaaga agcagataag ggaaatagcc acagtaaaaa 1680
   aatgcttaaa gcacttctaa gtgagggtga aagcatctgg gagatcactg agaaaatact 1740
   aaattcgttt gagtatacct cgagatttac aaaaacaaaa actttatacc aattcctctt 1800
   cctagctact ttcatcaatt gtggaagatt cagcgatatt aagaacgttg atccgaaatc 1860
   atttaaatta gtccaaaata agtatctggg agtaataatc cagtgtttag tgacagagac 1920
   aaagacaagc gttagtaggc acatatactt ctttagcgca aggggtagga tcgatccact 1980
   tgtatatttg gatgaatttt tgaggaattc tgaaccagtc ctaaaacgag taaataggac 2040
   cggcaattct tcaagcaaca aacaggaata ccaattatta aaagataact tagtcagatc 2100
   gtacaacaag gctttgaaga aaaatgcgcc ttatccaatc tttgctataa agaatggccc 2160
   aaaatctcac attggaagac atttgatgac ctcatttctg tcaatgaagg gcctaacgga 2220
   gttgactaat gttgtgggaa attggagcga taagcgtgct tctgccgtgg ccaggacaac 2280
   gtatactcat cagataacag caatacctga tcactacttc gcactagttt ctcggtacta 2340
   tgcatatgat ccaatatcaa aggaaatgat agcattgaag gatgagacta atccaattga 2400
   ggagtggcag catatagaac agctaaaggg tagtgctgaa ggaagcatac gataccccgc 2460
   atggaatggg ataatatcac aggaggtact agactacctt tcatcctaca taaatagacg 2520
   catataataa tctagagctc gctgatcagc ctcgactgtg ccttctagtt gccagccatc 2580
   tgttgtttgc ccctcccccg tgccttcctt gaccctggaa ggtgccactc ccactgtcct 2640
   ttcctaataa aatgaggaaa ttgcatcgca ttgtctgagt aggtgtcatt ctattctggg 2700
   gggtggggtg gggcaggaca gcaaggggga ggattgggaa gacaatagca ggcatgctgg   2760
   ggatgcggtg ggctctatgg cttctgaggc ggaaagaacc agctggggct cgagggggga   2820
   tcccgtcgac ctcgagagct tggcgtaatc atggtcatag ctgtttcctg tgtgaaattg 2880
   ttatccgctc acaattccac acaacatacg agccggaagc ataaagtgta aagcctgggg 2940
   tgcctaatga gtgagctaac tcacattaat tgcgttgcgc tcactgcccg ctttccagtc 3000
   gggaaacctg tcgtgccagc tgcattaatg aatcggccaa cgcgcgggga gaggcggttt 3060
   gcgtattggg cgctcttccg cttcctcgct cactgactcg ctgcgctcgg tcgttcggct 3120
   gcggcgagcg gtatcagctc actcaaaggc ggtaatacgg ttatccacag aatcagggga 3180
   taacgcagga aagaacatgt gagcaaaagg ccagcaaaag gccaggaacc gtaaaaaggc 3240
   cgcgttgctg gcgtttttcc ataggctccg cccccctgac gagcatcaca aaaatcgacg 3300
   ctcaagtcag aggtggcgaa acccgacagg actataaaga taccaggcgt ttccccctgg 3360
   aagctccctc gtgcgctctc ctgttccgac cctgccgctt accggatacc tgtccgcctt 3420
   tctcccttcg ggaagcgtgg cgctttctca atgctcacgc tgtaggtatc tcagttcggt 3480
   gtaggtcgtt cgctccaagc tgggctgtgt gcacgaaccc cccgttcagc ccgaccgctg 3540
   cgccttatcc ggtaactatc gtcttgagtc caacccggta agacacgact tatcgccact 3600
   ggcagcagcc actggtaaca ggattagcag agcgaggtat gtaggcggtg ctacagagtt 3660
   cttgaagtgg tggcctaact acggctacac tagaaggaca gtatttggta tctgcgctct 3720
   gctgaagcca gttaccttcg gaaaaagagt tggtagctct tgatccggca aacaaaccac 3780
   cgctggtagc ggtggttttt ttgtttgcaa gcagcagatt acgcgcagaa aaaaaggatc 3840
   tcaagaagat cctttgatct tttctacggg gtctgacgct cagtggaacg aaaactcacg 3900
   ttaagggatt ttggtcatga gattatcaaa aaggatcttc acctagatcc ttttaaatta 3960
   aaaatgaagt tttaaatcaa tctaaagtat atatgagtaa acttggtctg acagttacca 4020
   atgcttaatc agtgaggcac ctatctcagc gatctgtcta tttcgttcat ccatagttgc 4080
   ctgactcccc gtcgtgtaga taactacgat acgggagggc ttaccatctg gccccagtgc 4140
   tgcaatgata ccgcgagacc cacgctcacc ggctccagat ttatcagcaa taaaccagcc 4200
   agccggaagg gccgagcgca gaagtggtcc tgcaacttta tccgcctcca tccagtctat 4260
   taattgttgc cgggaagcta gagtaagtag ttcgccagtt aatagtttgc gcaacgttgt 4320
   tgccattgct acaggcatcg tggtgtcacg ctcgtcgttt ggtatggctt cattcagctc 4380
   cggttcccaa cgatcaaggc gagttacatg atcccccatg ttgtgcaaaa aagcggttag 4440
   ctccttcggt cctccgatcg ttgtcagaag taagttggcc gcagtgttat cactcatggt 4500
   tatggcagca ctgcataatt ctcttactgt catgccatcc gtaagatgct tttctgtgac 4560
   tggtgagtac tcaaccaagt cattctgaga atagtgtatg cggcgaccga gttgctcttg 4620
   cccggcgtca atacgggata ataccgcgcc acatagcaga actttaaaag tgctcatcat 4680
   tggaaaacgt tcttcggggc gaaaactctc aaggatctta ccgctgttga gatccagttc 4740
   gatgtaaccc actcgtgcac ccaactgatc ttcagcatct tttactttca ccagcgtttc 4800
   tgggtgagca aaaacaggaa ggcaaaatgc cgcaaaaaag ggaataaggg cgacacggaa 4860
   atgttgaata ctcatactct tcctttttca atattattga agcatttatc agggttattg 4920
   tctcatgagc ggatacatat ttgaatgtat ttagaaaaat aaacaaatag gggttccgcg 4980
   cacatttccc cgaaaagtgc cacctgacgt cgacggatcg gga                   5023
<210> 4
   <211> 1024
   <212> DNA
   <213> Artificial
<220>
   <223> Nucleic acid fragment
<220>
   <221> misc_feature
   <222> (140)..(140)
   <223> n is a, c, g, or t
<220>
   <221> misc_feature
   <222> (160)..(160)
   <223> n is a, c, g, or t
<400> 4
   tggaaacaga aactaaatag agacatagag aaaatgaaca gaagttatga accaaatgga      60
   tttaaccggt atttatacaa catttcatct taaaacaaaa gaatatacct tcttctcagc     120
   acctcatagc ctcttctacn aaaccatata gtcggtcacn aaacaagcct caacagatac     180
   aagaagataa aaataatccc atgcatacta tcagatcacc atggactaac tctggtcttc     240
   aataacaaca aaaacaatgg aagtcgaaga acactctatt caatgataac ttggtcaagg     300
   aagagatgaa aaaggaaatt aaagcctttt tagaatttca tgaaaatgaa ggcacaacat     360
   acccaaactt atgagacaca aggaaagcag tgctaagagg aaaactcata gctctgagtg     420
   cctccaaaaa gaaacaagag agagtgtata ctagcagctt gagagcacac ctgaaagctc     480
   tagaacaaaa agaagcaaat acacccaaga ggagaagatc aaatgcaggg ctgaaatcaa     540
   ccaagtagaa acaaaagaac tgtaaaaaga accaacaaaa ccaggagttg gttggttgag     600
   aaaatcaaaa agataaataa acccttaatc agaataacca gagggcacag aaacagtatc     660
   caaattaaca aaataagaaa tggaaaggga gacgtaaaac agaatccgag gaaataaaaa     720
   aaaattgatc ctattacaaa agtctatatt caacaaagct ggaaaatctg gatgaaatag     780
   acaattttct agatagatac cagataccaa agttaaatca ggaccagata aatcatctaa     840
   acagtcccat aactcctaaa gaaatagaag cagttattaa aattctcccg accaaaaaaa     900
   aaaaaaaaaa aaagcccagg attggatggg tttagtggag aattctatca gaccttcatc     960
   aaagacctaa taccaatact gtccaaacta ttccttgcaa cactgaatat tccgcataca    1020
   ctat                                                                 1024
<210> 5
   <211> 50
   <212> DNA
   <213> Artificial
<220>
   <223> Primer
<400> 5
   acagctgtcg actgaagtac ctattccgaa gttcctattc tctagaaagt 50
<210> 6
   <211> 50
   <212> DNA
   <213> Artificial
<220>
   <223> primer
<400> 6
   cgtccggata tctaagatct gaagttccta tactttctag agaataggaa 50
<210> 7
   <211> 81
   <212> DNA
   <213> Artificial
<220>
   <223> PCR product
<400> 7
   agacgtctcg actgaagtac ctattccgaa gttcctattc tctagaaagt ataggaactt 60
   cagatcttag atatccggac g 81
<210> 8
   <211> 40
   <212> DNA
   <213> Artificial
<220>
   <223> Primer
<400> 8
   atctggctag ccgccaccat gccacaattt gatatattat 40
<210> 9
   <211> 34
   <212> DNA
   <213> Artificial
<220>
   <223> Primer
<400> 9
   tgtcatctag attattatat gcgtctattt atgt 34
<210> 10
   <211> 22
   <212> DNA
   <213> Artificial
<220>
   <223> Primer
<400> 10
   cctatggaaa aacgccagca ac 22
<210> 11
   <211> 22
   <212> DNA
   <213> Artificial
<220>
   <223> Primer
<400> 11
   ccttagaaag cggtctgtga aa 22
<210> 12
   <211> 19
   <212> DNA
   <213> Artificial
<220>
   <223> Primer
<400> 12
   gatgcctgct tgccgaata 19
<210> 13
   <211> 22
   <212> DNA
   <213> Artificial
<220>
   <223> Primer
<400> 13
   ccttagaaag cggtctgtga aa 22
<210> 14
   <211> 23
   <212> DNA
   <213> Artificial
<220>
   <223> Primer
<400> 14
   tggtatggct gattatgatc ctc 23
<210> 15
   <211> 22
   <212> DNA
   <213> Artificial
<220>
   <223> Primer
<400> 15
   ccttttgctc acatggctcg ac 22
<210> 16
   <211> 20
   <212> DNA
   <213> Artificial
<220>
   <223> Primer
<400> 16
   ttgtctcatg agcggataca 20